(19) Europäisches Patentamt
European Patent Office
Office européen des brevets

(11) EP 2 307 041 B1

(12) **EUROPEAN PATENT SPECIFICATION**

(45) Date of publication and mention of the grant of the patent:
**13.03.2019 Bulletin 2019/11**

(21) Application number: **09759115.0**

(22) Date of filing: **29.05.2009**

(51) Int Cl.:
*A61K 38/10* (2006.01) *A61P 35/00* (2006.01)

(86) International application number:
**PCT/US2009/045658**

(87) International publication number:
**WO 2009/148954 (10.12.2009 Gazette 2009/50)**

(54) **TPO MIMETIC PEPTIDE FOR PREVENTING HEMATOLOGICAL DISORDER ASSOCIATED WITH CANCER TREATMENT**

TPO MIMETIC ZUR VORBEUGUNG HAEMATOLOGISCHER STÖRUNGEN, DIE MIT EINER KREBSBEHANDLUNG EINHERGEHEN

TPO MIMETIQUE POUR PREVENIR DES TROUBLES HÉMATOLOGIQUES ASSOCIES AU TRAITEMENT DU CANCER

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **03.06.2008 US 58376 P**

(43) Date of publication of application:
**13.04.2011 Bulletin 2011/15**

(73) Proprietor: **Janssen Pharmaceutica, N.V.**
**2340 Beerse (BE)**

(72) Inventors:
- **YURKOW, Edward, J.**
**Hillsborough**
**NJ 08844 (US)**
- **SHUKLA, Umesh**
**Belle Mead**
**NJ 08502 (US)**

(74) Representative: **V.O.**
**P.O. Box 87930**
**2508 DH Den Haag (NL)**

(56) References cited:
**WO-A1-2007/094781** **US-A1- 2006 040 866**
**US-A1- 2008 119 384**

- **DE SERRES M ET AL: "Pharmacokinetics and hematological effects of the PEGylated thrombopoietin peptide mimetic GW395058 in rats and monkeys after intravenous or subcutaneous administration" STEM CELLS, ALPHAMED PRESS, DAYTON, OH, US, vol. 17, no. 6, 1 January 1999 (1999-01-01), pages 316-326, XP002421200 ISSN: 1066-5099**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

RELATED APPLICATION

FIELD OF THE INVENTION

The present invention provides a method for treating and/or preventing hematological disorders such as anemia and thrombocytopenia in a subject undergoing treatment for cancer whereby a TPO mimetic peptide compound is administered using a specified dosing regimen. The dosing regimen involves the administration of the TPO mimetic peptide compound within a specified time frame surrounding administration of a chemotherapeutic agent. The dosing regimen also involves monitoring the subject's hematological parameters in order to determine the dose for subsequent treatments.

BACKGROUND OF THE INVENTION

Anemia

Approximately 75% of cancer subjects receiving chemotherapy develop anemia, and the severity and incidence of anemia increases as the treatment cycles increase. The incidence of anemia was highest in subjects with lung cancer (83.3%) and gynecological malignancies (88.3%).1 Similar incidence has been reported in an earlier publication, as summarized in Table 1.[2] The severity and incidence of anemia depends on a number of factors, including the type and extent of disease, and type, schedule, intensity, and duration of chemotherapy. For example, the incidence of Grade 2 or higher anemia has been reported to be as high as 71% in non-small cell lung cancer (NSCLC) subjects receiving platinum analogs and gemcitabine combination and the incidence of Grade 3 or higher anemia in this setting has been reported to be 28% (literature reported range from 5 to 28%).[3,4] The incidence of transfusion in this setting has been reported to be approximately 39%.[5]

**Table 1. Estimated Incidence and Severity of Chemotherapy-Induced Anemia**

| Advanced Tumor Type | Estimated New Cases 2005 (US) | Grade 1-2 Anemia (Hb >8 g/dL) | Grade 3-4 Anemia (Hb <8 g/dL) |
|---|---|---|---|
| Non-Small-Cell Lung Cancer | 155,000 | up to 85% | up to 34% |
| Small-Cell Lung Cancer | 17,000 | up to 75% | up to 55% |
| Breast | 213,000 | up to 84% | up to 11% |
| Ovarian | 22,000 | up to 78% | up to 42% |
| Lymphomas | 63,000 | up to 63% | up to 79% |
| Colorectal | 150,000 | up to 60% | up to 10% |
| Head and Neck | 29,000 | up to 74% | up to 14% |
| Hb = hemoglobin | | | |

Fatigue is one of the major symptoms associated with chemotherapy induced anemia (CIA). Anemia is well recognized as an adverse prognostic factor for many cancers. Anemia has been reported to be negatively associated with survival in a wide variety of cancers such as lung, head and neck, myeloma, prostate and lymphoma.[6] Prior to the advent of the erythropoiesis stimulating agents (ESAs) (epoetin alfa (Epogen®, Eprex®, Procrit®), epoetin beta (Neo Recormon®), and darbepoetin alfa (Aranesp®)), treatment of CIA was limited to severe cases of anemia (Hb levels of 7-8 g/dL or less) due to dependence on donor red blood cell transfusions, which are a limited resource and are associated with concerns over transmission of infectious diseases and alloimmunization. ESAs have changed the treatment strategy for CIA. Several organizations have developed clinical practice guidelines for the use of ESAs, based upon published studies demonstrating increases in Hb levels, decreases in transfusion requirements, and improvements in quality of life.[7-9] The American Society of Clinical Oncology/American Society of Hematology (ASCO/ASH) guidelines revised in 2007 recommend initiating ESA treatment as hemoglobin (Hb) approaches, or falls below, 10 g/dL.[10] However, current use of ESAs has been associated with possible increased thrombotic vascular events and increased mortality in hemodialysis subjects with cardiac disease, subjects undergoing coronary artery bypass surgery or breast cancer subjects receiving chemotherapy.[11]

An agent that could prevent CIA from occurring at the initiation of chemotherapy treatment without increasing

the Hb levels above baseline, and thus eliminating potentially harmful Hb increases known to be associated with ESAs, is highly desirable. Thrombopoietin (TPO) is the primary physiologic regulator of platelet production but additional evidence indicates that TPO has a more pleiotropic range of activities. Pancytopenia has been observed in the absence of TPO or its receptor, c-mpl.[12,13] TPO maintains hematopoietic stem cell viability,[14,15] prevents apoptosis of irradiated bone marrow cells,[16] causes expansion of the stem cell population in combination with other cytokines,[17] enhances in vivo platelet and erythroid recovery following irradiation,[18] and enhances stem cell mobilization into peripheral blood.[19] These multilineage effects support the hypothesis that a TPO agonist could effectively ameliorate CIA by limiting apoptosis in multipotential hematopoietic progenitor cells and by expanding the hematopoietic stem cell population.

Thrombocytopenia

In addition, a TPO agonist could be efficacious in preventing chemotherapy-induced thrombocytopenia (CIT). Subjects with CIT can have clinically significant bleeding episodes, which are associated with poor clinical outcomes. Such bleeding episodes lead to delay of chemotherapy or dose modification.[20]

The TPO Mimetic Peptide Compound

The TPO mimetic peptide compound is a PEGylated TPO mimetic peptide that has no homology with TPO and the potential to prevent CIA and CIT. See, e.g., US 7,576,056, US 7,723,295, US 2008/0119384 (U.S.

Patent Applications Serial Nos. 10/918,561, filed August 13, 2004; 11/200,416, filed August 9, 2005; and 11/354,065, filed February 14, 2006,). The lack of homology with TPO reduces the potential for generation of anti TPO antibodies. The PEGylation of the peptide leads to a reduced clearance of the compound without loss of potency.

The TPO mimetic peptide compound is a 29-mer peptide having two identical 14-mers linked by a lysinamide residue as follows:

I E G P T L R Q (2-Nal) L A A R (Sar)
\
K($NH_2$)
/
I E G P T L R Q (2-Nal) L A A R (Sar)

having a 20,000 MPEG residue covalently linked to each N-terminal isoleucine. The full molecular structure of the TPO mimetic peptide compound is detailed below:

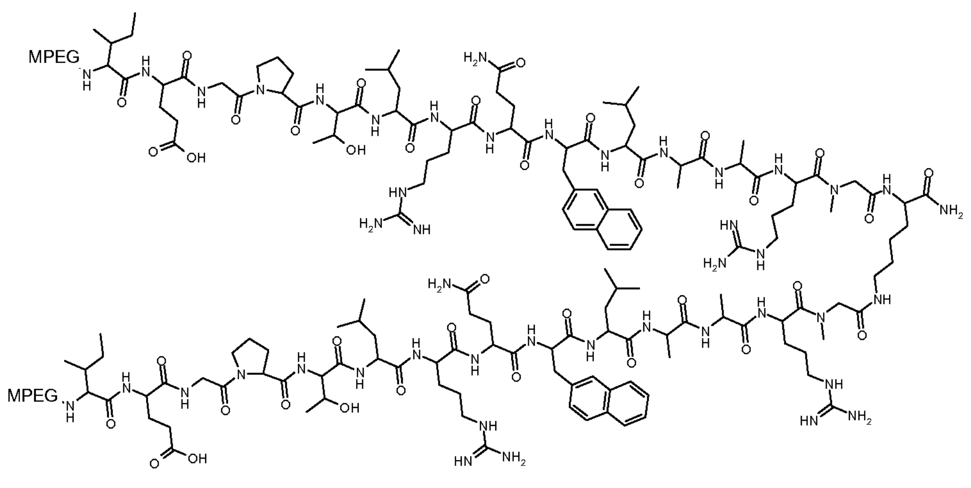

The full chemical name of the TPO mimetic peptide compound is: Methoxypolyethyleneglycol20000-propionyl-L-Isoleucyl-L-Glutamyl-Glycyl-L-Prolyl-L-Threonyl-L-Leucyl-L-Arginyl-L-Glutaminyl-L-2-Naphthylalanyl-L-Leucyl-L-Alanyl-L-Alanyl-L-Arginyl-Sarcosyl-Ne-(methoxypolyethyleneglyco120000-propionyl-L-Isoleucyl-L-Glutamyl-Glycyl-L-Prolyl-L-Threonyl-L-Leucyl-L-Arginyl-L-Glutaminyl-L-2-Naphthylalanyl-L-Leucyl-L-Alanyl-L-Alanyl-L-Arginyl-Sarcosyl-)-Lysinamide.

The TPO mimetic peptide compound is thus composed of two identical 14 amino acid peptide chains linked by a lysinamide residue and linked on each N-terminal to an approximately 20,000 Dalton molecular weight polyethylene glycol (PEG) chain. The molecular weight of the parent peptide without PEG is 3,295 Daltons and with two PEG chains is approximately 43,295 Daltons. The TPO mimetic peptide compound has an abbreviated molecular structure of (MPEG-Ile-Glu-Gly-Pro-Thr-Leu-Arg-Gln-(2-Nal)-Leu-Ala-Ala-Arg-(Sar))$_2$-Lys-$NH_2$; where (2-Nal) is β-(2-naphthyl)alanine, (Sar) is sarcosine and MPEG is methoxypoly(ethylene glycol) (MW approximately 20,000 Daltons).

Pre-Clinical Studies

Pre-clinical studies with the TPO mimetic peptide compound demonstrated an effect on carboplatin induced anemia and carboplatin induced thrombocytopenia in mice. See, e.g., US 7,576,056, US 7,723,295, US 2008/0119384 (U.S. Patent Applications Nos. 10/918,561, filed August 13, 2004; 11/200,416, filed August 9, 2005; and 11/354,065, filed February 14, 2006). The data supported a potential myeloprotective and lineage stimulation mechanism. One potential concern with the use of growth factors in cancer treatment has been the potential for promotion of tumor growth. The TPO mimetic peptide compound administration alone did not enhance tumor growth and the addition of up to 3 cycles of the TPO mimetic peptide compound treatment to carboplatin therapy had no negative impact on tumor growth delay and in fact led to a small advantage in survival when compared with carboplatin treatment alone. See Example 1. These results are consistent with the literature reports that the thrombopoietin receptor c-mpl has an extremely limited expression in tumor cell lines of the non-myeloid lineage.[22] Clinical Studies in Healthy Volunteers

The first in human study demonstrated that single intravenous (i.v.) doses of the TPO mimetic peptide compound were safe and generally well tolerated over the dose range tested (0.375 to 3 μg/kg), and there was no evidence of antibody formation against the TPO mimetic peptide compound. See, e.g., US 2008/0119384 (U.S. Patent Application No. 11/354,065, filed February 14, 2006,). A dose dependent, nonlinear increase in mean platelet counts was observed. There were also indications that the TPO mimetic peptide compound increased mean numbers of hematopoietic progenitor cells, although the study was not specifically designed to evaluate this.

Clinical Studies in Cancer Subjects

Preliminary results from an ongoing study in 46 subjects with cancer (N=12 at 1.5 μg/kg, 12 at 2.25 μg/kg, 10 at 3 μg/kg, and 12 placebo) receiving platinum based chemotherapy suggest that, as compared with placebo, 2.25 and 3 μg/kg doses of the TPO mimetic peptide compound increased platelet count and showed trends for preservation of hemoglobin. See Example 2. These data suggest that the TPO mimetic peptide compound has potential utility in prevention of chemotherapy-induced anemia and thrombocytopenia.

In subjects with NSCLC receiving a 21-day chemotherapy regimen of gemcitabine and either carboplatin or cisplatin, co-administration of the TPO mimetic peptide compound provides a lower incidence rate of (1) the composite endpoint of Grade 2 or higher anemia, or (2) a ≥ 2 g/dL drop in hemoglobin on the first day of any chemotherapy cycle (Cycle 2 to 6) relative to baseline (Cycle 1, Day 1), or (3) the use of rescue intervention for anemia (e.g., erythropoiesis stimulating agents [ESAs], red blood cell [RBC] transfusion) as compared to placebo.

In subjects with NSCLC receiving a 21-day chemotherapy regimen of gemcitabine and either carboplatin or cisplatin, co-administration of the TPO mimetic peptide compound provides a lower incidence rate of (1) the composite endpoint of Grade 2 or higher thrombocytopenia or (2) the use of platelet transfusion as compared to placebo.

An increased platelet count of > 3 times baseline or above 1,000,000 platelets per μL in plasma are considered excessive and provide an increased risk of thrombovascular events in subjects with cancer. Platelets, particularly when activated, are a key factor in a coagulation cascade that results in thrombus formation. A need thus exists for a dosing regimen for the TPO mimetic peptide compound that takes into account the platelet results of each individual patient during each cycle of chemotherapy, and that minimizes or overcomes the risk of thrombovascular events due to excessive platelet increases while treating the patients with the TPO mimetic peptide compound for prevention of CIA and/or CIT.

The dosing regimen of the invention was designed to increase the safety of the TPO mimetic peptide compound and to increase the efficacy of the TPO mimetic peptide compound while treating and/or preventing CIA and CIT in subjects undergoing chemotherapy.

SUMMARY OF THE INVENTION

The invention includes a method for treating and/or preventing chemotherapy induced anemia in a subject undergoing treatment for cancer, comprising administering the TPO mimetic peptide compound as defined in claim 1 within two hours prior to said administration of said cancer treatment.

The invention is based, in part, on the determination that the maximum effect of the TPO mimetic peptide

compound on platelet count occurs on Day 15 after administration of said TPO mimetic peptide compound.

In a preferred embodiment, platelet count is determined.

In another preferred embodiment, hemoglobin value is determined.

Further described is adjusting the dose of the TPO mimetic peptide compound, if necessary, based on the subject's hematological values. Adjusting the dose includes reducing the dose or withholding the dose.

As a means of exemplifying this aspect of the invention, the TPO mimetic peptide compound doses for each cycle of a six cycle treatment regimen are described in Table 2.

Dosing based on platelet response

**Table 2**

| **Overview of The TPO Mimetic Peptide Compound Doses for Cycle 1 to 6** | |
|---|---|
| **Cycle** | **The TPO Mimetic Peptide Compound Dose (μg/kg)** |
| 1 | 2.5 |
| 2 | 3.0[1] |
| 3 | 2.0 to 3.5[2] |
| 4 | 2.0 to 3.5[2] |
| 5 | 2.0 to 3.5[3] |
| 6 | 2.0 to 3.5[2] |

[1]If necessary, the dose will be reduced to 2.5 μg/kg or withheld based on the subject's platelet count on Cycle 1, Day 15 and Cycle 2, Day 1.

[2] If necessary, the dose will be titrated or withheld based on the subject's platelet count on Day 15 of the preceding Cycle and Day 1 of this Cycle.

On Day 1 in Cycle 2, each subject will receive 3.0 μg/kg of the TPO mimetic peptide compound. In the event a subject's Cycle 1 Day 15 platelet count is >700,000/μL, and the platelet count remains >500,000/μL but is < 700,000/μL on Day 1 of Cycle 2, the subject will receive a reduced dose of 2.5 μg/kg of the TPO mimetic peptide compound or placebo. If the platelet count is > 700,000/μL on Day 1 of Cycle 2, the subject will not be dosed with the TPO mimetic peptide compound in Cycle 2.

As indicated in the Table 3, on Day 1 of Cycle 3 to 6, the dose of the TPO mimetic peptide compound will be based on the subject's Day 15 platelet count in the previous cycle.

In Cycle 2 to 5, if a subject's Day 15 platelet count is > 700,000/μL, and the platelet count on Day 1 of the next chemotherapy cycle remains > 700,000/μL, the subject will not be dosed with the TPO mimetic peptide compound or placebo for that given cycle. Subjects will be dosed again for subsequent cycles if platelets are < 700,000/μL on Day 1 of that chemotherapy cycle.

As a means of exemplifying this aspect of the invention, a detailed dose titration scheme is provided below:

**Table 3. The TPO Mimetic Peptide Compound Dose Titration Scheme for Cycle 3 to 6**

| **Day 15 platelet count of previous chemotherapy cycle (Cycle 2 to 5) x 1000 (/μL)** | **The TPO mimetic peptide compound dose (μg/kg) for next chemotherapy cycle (Cycle 3 to 6)** |
|---|---|
| ≥ 900 [1] | 2.0 |
| 501 - 899 [1] | 2.5 |
| 101 - 500 | 3.0 |
| 50 - 100 | 3.25 |
| < 50 | 3.5 |

(continued)

| Day 15 platelet count of previous chemotherapy cycle (Cycle 2 to 5) x 1000 (/μL) | The TPO mimetic peptide compound dose (μg/kg) for next chemotherapy cycle (Cycle 3 to 6) |
|---|---|
| [1] If the subject's platelet count on Day 1 of the next chemotherapy cycle is > 700,000/μL, the subject will not be dosed with the TPO mimetic peptide compound or placebo for that given cycle. Subjects could be dosed again for subsequent cycles if platelets are < 700,000/μL on Day 1 of that chemotherapy cycle. If platelet count continues to be > 700,000/μL on Day 1 of two consecutive cycles, the subject will not be given additional doses of the TPO mimetic peptide compound. | |

Dosing based on hemoglobin value

In order to further maximize subject safety, hemoglobin values for each subject will also be evaluated on Day 1 of each chemotherapy cycle to determine if the dose of the TPO mimetic peptide compound should be held. Specifically, if a subject has a hemoglobin value >15 g/dL or has an increase from baseline of ≥ 2 g/dL on Day 1 of any cycle, the subject will not be dosed with the TPO peptide mimetic compound for that given cycle. The dose of the TPO mimetic peptide compound will not be modified based on hemoglobin values.

The TPO mimetic peptide compound or placebo will be administered as an IV bolus on Day 1 of each chemotherapy cycle, within 2 hours prior to receiving chemotherapy.

**EFFICACY EVALUATIONS**

Efficacy evaluations include:

The difference in incidence rates between the TPO mimetic peptide compound and placebo on (1) the composite endpoint of Grade 2 or higher anemia, or (2) a ≥ 2 g/dL drop in hemoglobin on the first day of any chemotherapy cycle (Cycle 2 to 6) relative to baseline (Cycle 1, Day 1), or (3) the use of rescue intervention (e.g., ESAs, RBC transfusion) for anemia may be employed for efficacy evaluation.

The difference between the TPO mimetic peptide compound and placebo on the incidence rates on the composite endpoint of Grade 2 or higher thrombocytopenia or the use of platelet transfusion.

The difference between the TPO mimetic peptide compound and placebo on the incidence rates of each individual component of the composite endpoints for anemia and thrombocytopenia.

Hemoglobin, platelet count, use of ESAs, and the use of RBC and platelet transfusions may be the criteria used to evaluate the efficacy endpoints. These parameters may also be part of the safety evaluation.

BRIEF DESCRIPTION OF THE DRAWINGS

Figure 1 is a graph showing the individual times to endpoint by group for all animals studied in Example 1.

Figure 2 is a graph showing the median tumor growth curves (Figure 2a) and Kaplan-Meier plots (Figure 2b) for the animal groups studied in Example 1.

Figures 3a - 3c show mean platelet, reticulocyte and hematocrit values, respectively, for Groups 6-9 of animals studied in Example 1 on Days 10, 13, 21, and 24. These data are also included in tabular form in Tables 4a - 4d.

Figure 4 is a graph showing Mean Change of Platelet Counts from Baseline (Mean +/- SE) as a result of treatment of subjects with the TPO mimetic peptide compound in accordance with Example 2.

Figure 5 is a graph showing Least Squares Mean Change of Hemoglobin Values From Baseline (Mean +/- SE) as a result of treatment of subjects with the TPO mimetic peptide compound in accordance with Example 2.

DETAILED DESCRIPTION OF THE INVENTION

Abbreviations

The following abbreviations may be used throughout the specification.

| | |
|---|---|
| AE(s) | Adverse event(s) |
| ANC | Absolute neutrophil count |
| aPTT | Activated partial thromboplastin time |
| AST | Aspartate aminotransferase |
| BFI | Brief Fatigue Inventory |
| BFU-E | Burst-forming unit-erythroid |
| CIA | Chemotherapy-induced anemia |
| CIT | Chemotherapy-induced thrombocytopenia |
| D-dimer | Fibrin split product, D-dimer |
| ECG | Electrocardiogram |
| EC50 | Effective concentration inducing 50% maximal effect |
| ECOG | Eastern Cooperative Oncology Group |
| ESA | Erythropoiesis-stimulating agent |
| FACT-An | Functional Assessment of Cancer Therapy-Anemia |
| F1+2 | Prothrombin fragment F1+2 |
| FU | Follow up |
| GIC | Global impression of change |
| Hb | Hemoglobin |
| huEPO | Human erythropoietin |
| huTPO | Human thrombopoietin |
| IL | Interleukin |
| iv | Intravenous |
| LLN | Lower limit of normal range |
| LS | Least squares |
| NSCLC | Non-small cell lung cancer |
| PD | Pharmacodynamic |
| PDGF | Platelet derived growth factor |
| PEG | Polyethylene glycol |
| PF4 | Platelet Factor 4 |
| PFS | Progression-free survival |
| PK | Pharmacokinetic |
| RBC | Red blood cell |
| RECIST | Response Evaluation Criteria in Solid Tumors |
| TGF | Transforming growth factor |
| TIBC | Total iron binding capacity |
| TPO | Thrombopoietin |
| TVE | Thrombovascular event |
| WBC | White blood cell |
| WNL | Within normal limits |

Definitions

The following defined terms may be used throughout the specification.

As used herein, the terms "comprising", "containing", "having" and "including" are used in their open, non-limiting sense.

"Anemia" is a deficiency of red blood cells (RBCs) and/or hemoglobin. This results in a reduced ability of blood to transfer oxygen to the tissues, causing tissue hypoxia. Since all human cells depend on oxygen for survival, varying degrees of anemia can have a wide range of clinical consequences. Hemoglobin (the oxygen-carrying protein in the red blood cells) has to be present to ensure adequate oxygenation of all body tissues and organs.

"Grade of anemia" is the severity of anemia on a scale from 0 to 5 as determined in accordance with criteria specified in Attachment 1.

"Grade of thrombocytopenia" is the severity of thrombocytopenia on a scale from 0 to 5 as determined in accordance with criteria specified in Attachment 1.

"Hematocrit" (Ht or HCT) and packed cell volume (PCV) are measures of the proportion of blood volume that is occupied by red blood cells. It is normally 45 $\pm$ 7 (38-52%) for males and 42 $\pm$ 5 (37-47%) for females.

"Hemoglobin", also spelled haemoglobin and abbreviated Hb, is the iron-containing oxygen-transport metalloprotein in the red blood cells of the blood.

"Hemoglobin value" is the amount of hemoglobin in blood in g/dL.

Mean platelet volume" (MPV) is a measurement of the average size of platelets found in blood and is typically included in blood tests. Since the average platelet size is larger when the body is producing increased numbers of platelets, MPV test results can be used to make inferences about platelet production in bone marrow.

"Myelosuppressive agent" is an agent, which causes a condition in which bone marrow activity is decreased, resulting in fewer red blood cells, white blood cells, and platelets.

"Nadir" is the lowest blood count for a given patient in a given period of time (i.e., a patient's ANC Nadir or absolute neutrophil count). For example, patients undergoing chemotherapy will exhibit an ANC Nadir a week after starting therapy due to bone marrow suppression.

"Neutropenia" is a hematological disorder characterized by an abnormally low number of neutrophils (a type of white blood cell). Neutrophils usually make up 50-70% of circulating white blood cells and serve as the primary defense against infections by destroying bacteria in the blood. Hence, patients with neutropenia are more susceptible to bacterial infections and without prompt medical attention, the condition may become life-threatening.

"Neutrophil count" otherwise know as "absolute neutrophil count" (ANC) is a measure of the number of neutrophil granulocytes (also known as polymorphonuclear cells, PMN's, polys, granulocytes, segmented neutrophils or segs) present in the blood. Neutrophils are a type of white blood cell that fights against infection. The ANC is calculated from measurements of the total number of white blood cells (WBC) and the numbers of neutrophils and bands, which form a subset of the total number of white blood cells. A normal ANC is above 1,500. An ANC less than 500 is defined as neutropenia and significantly increases the risk of infection. Neutropenia is the condition of a low ANC, and the most common condition where an ANC would be measured is in the setting of chemotherapy for cancer.

"Pancytopenia" is a medical condition in which there is a reduction in the number of red and white blood cells, as well as platelets. Pancytopenia is generally due to diseases affecting the bone marrow. Chemotherapy for malignancies may also cause pancytopenia, if the drug or drugs used cause bone marrow suppression.

"Platelet count" is the calculated number of platelets in a volume of blood, usually expressed as platelets per cubic millimeter (cmm) of whole blood. Normal platelet counts are in the range of about 150,000 to 450,000 per microliter (or 150 - 450 x $10^9$ per liter). These values many vary slightly between different laboratories.

"Red blood cells" otherwise know as "erythrocytes" are the most common type of blood cell and the principal means of delivering oxygen from the lungs to body tissues via the blood.

"Thrombocytopenia" is the presence of relatively few platelets in blood. Generally speaking a normal platelet count ranges from about 150,000 and 450,000 per $mm^3$. These limits, however, are determined by the $2.5^{th}$ lower and upper percentile, and a deviation does not necessarily imply any form of disease. The number of platelets in a blood sample also decreases rather quickly with time and a low platelet count may be caused by a delay between sampling and analysis.

"White blood cell count" is the number of white blood cells (WBCs) in the blood. The WBC is usually measured as part of the CBC (complete blood count). White blood cells are the infection-fighting cells in the blood. There are different types of white blood cells, including neutrophils (polymorphonuclear leukocytes; PMNs), band cells (slightly immature neutrophils), T-type lymphocytes (T cells), B-type lymphocytes (B cells), monocytes, eosinophils, and basophils. All the types of white blood cells are reflected in the white blood cell count. The normal range for the white blood cell count varies between laboratories but is usually between 4,300 and 10,800 cells per cubic millimeter of blood. This can also be referred to as the leukocyte count and can be expressed in international units as 4.3 - 10.8 x $10^9$ cells per liter.

## Non-clinical Pharmacology

The TPO mimetic peptide compound has an estimated $EC_{50}$ of approximately 5 pM (0.2 ng/mL) in a human TPO (huTPO) receptor assay in vitro and stimulates megakaryocyte lineage specific growth and differentiation in vivo. A single i.v. dose of the TPO mimetic peptide compound (30 to 300 μg/kg) resulted in an increased platelet count in the rat that was maximal after 6 days and returned to baseline after 12 days. Additionally, a single dose of the TPO mimetic peptide compound displayed a myeloprotective effect in murine models of CIT by reducing the severity and duration of CIT in a dose-dependent manner (minimum effective dose 100 μg/kg) on Day 12.

Administration of the TPO mimetic peptide compound 1 hour after chemotherapy was more effective than dosing after 24 or 96 hours. In these studies, the TPO mimetic peptide compound also prevented a chemotherapy-induced reduction in Hb, hematocrit and RBC count, supporting a pluripotent protective effect on the megakaryocyte and erythroid lineages. Furthermore, following carboplatin treatment, the anti-anemic effect of the TPO mimetic peptide compound was observed at doses as low as 30 μg/kg. Additional studies also demonstrated that inhibition of chemotherapy-induced anemia and thombocytopenia by the TPO mimetic peptide compound correlated with a marked reduction in fibrinogen-positive microangiopathic lesions in small blood vessels in the brain. These histological findings suggest that prevention of the development of the microangiopathic events with the TPO mimetic peptide compound may be due to decreased platelet deposition as well as reduced microhemorrhage, which may contribute to prevention of chemotherapy-induced thrombocytopenia and anemia.

Example 1 (not part of the invention)

Administration of the TPO Mimetic Peptide Compound One Hour After Administration of Carboplatin. Effect on activity and toxicity of carboplatin against HT-29 human colon carcinoma xenografts established in athymic nude mice.

Five groups (n=10) of female athymic nude mice (Harlan) bearing established (~ 100 $mm^3$) HT-29 human colon carcinomas on Day 1. Groups 6-9 (n=20) were included for blood sampling and received the same treatment as Groups 1-4, respectively. Treatment effects on the growth of tumors were evaluated by tumor growth delay (TGD), which is the difference between median time to endpoint (TTE) tumor size in a treatment group compared to a control group. The effect of these treatments on platelet and erythrocyte precursors was determined from CBC analysis and reticulocyte counts of blood samples taken on Days 10, 13, 21 and 24.

**Table 4**

| Group # | Treatment | % Tumor Growth Delay | Median TTE in days | Tumor Burden Median | ? |
|---|---|---|---|---|---|
| Group 1, 6 | Untreated tumor controls | --- | 24.8 | --- | --- |
| Group 2, 7 | 60 mg/kg carboplatin, administered i.p. Days 1, 2, 12 and 13 | 92 | 47.6 | 600(1) | -3/1 %, Day 17 |
| Group 3, 8 | 0.2 mg/kg TPO mimetic peptide compound, administered i.v. Days 2, 13 and Days 2, 13 and 23, administered i.v. 1 hour after carboplatin dosing | 33 | 32.9 | --- | --- |
| Group 4, 9 | Combination of carboplatin and TPO mimetic peptide compound administered as above (Days 2, 13) | 119 | 54.2 | --- | -5.1 %, Day 17 |
| Group 5 | Combination of carboplatin and TPO mimetic peptide compound administered as above (Days 2, 13 and 23) | 150 | 62 | 877(?) | -3.5 %, Day 17 |

**TUMOR IMPLANTATION**

Xenografts were initiated from HT-29 human colon carcinoma xenografts maintained in athymic nude mice. HT-29 tumor fragments (1 $mm^3$) were implanted subcutaneously into the right flank of each test mouse, and tumor growth was monitored. On Day 1 of the study, the animals were pair-matched into five groups (Groups 1-5) for evaluation of efficacy and four groups for sampling (Groups 6-9). Groups 1-5 each consisted of ten animals with tumor sizes ranging from 75-126 $mm^3$ and group mean tumor sizes of 99 $mm^3$. Groups 6-9 each consisted of twenty animals with tumor sizes ranging from 40-221 $mm^3$ and group mean tumor sizes of 84 $mm^3$. Tumor weight was estimated with the assumption that 1 mg is equivalent to 1 $mm^3$ of tumor volume. Volume was calculated using the formula:

$$\text{Tumor Volume (mm}^3\text{)} = \frac{w^2 x\, l}{2}$$

where *w* = width and *l* = length in mm of an HT-29 tumor.

**THERAPEUTIC AGENT**

The TPO mimetic peptide compound was provided in nine tubes each containing 20 μL of a 10 mg/mL stock solution and stored at -20°C. Dosing solutions of the TPO mimetic peptide compound (20 μg/mL) were prepared fresh daily by transferring a 20 μL aliquot of stock solution under sterile conditions to 10 mL sterile saline and tumbling gently to mix. These mixtures were not allowed to foam and were not filter sterilized. Any residual dosing solutions and unused aliquots were stored at -20°C. Carboplatin (Sigma, Lot #034K0868) in powder form (three vials each containing 250 mg) was stored at room temperature. Carboplatin dosing solutions (6 mg/mL) were prepared fresh on day of dosing in sterile

phosphate buffered saline (PBS), and were filter sterilized prior to administration.

**TREATMENT**

Table 4 summarizes the treatment plan for this study. Group 1 mice (n = 10) were untreated tumor growth controls. Group 2 mice (n = 10) received 60 mg/kg carboplatin administered intraperitoneally (i.p.) on Days 1, 2, 12, and 13. Group 3 mice (n = 10) received 0.2 mg/kg of the TPO mimetic peptide compound administered intravenously (i.v.) via tail vein on Days 2 and 13. Groups 4 and 5 received the combination of carboplatin (60 mg/kg i.p. on Days 1, 2, 12, and 13) and 0.2 mg/kg of the TPO mimetic peptide compound on Days 2, 13 and Days 2, 13, 23, respectively, administered intravenously (i.v.) one hour after carboplatin dosing. Groups 6-9 (n = 20 each) were included for blood sampling and received the same treatments as Groups 1-4, respectively. For all groups, each dose of drug was given in a volume of 0.2 mL per 20g of body weight (10 mL/kg), and was scaled to the body weight of each animal.

**ENDPOINT**

Tumors were measured twice each week using calipers. Each animal was euthanized when its tumor reached the predetermined endpoint size of 1000 $mm^3$, or on the final day of the study (Day 62), whichever came first. The time to endpoint (TTE) for each mouse was calculated from the following equation:

$$\text{TTE (days)} = \frac{\log_{10}(\text{endpoint volume, mm}^3) - b}{m}$$

where b is the intercept and m is the slope of the line obtained by linear regression of a log-transformed tumor growth data set. The data set was comprised of the first observation that exceeded the study endpoint volume and the three consecutive observations that immediately preceded the attainment of the endpoint volume. Animals that did not reach the endpoint were assigned a TTE value equal to the last day of the study, animals classified as TR (treatment related) deaths were assigned a TTE value equal to the day of death, and animals classified as NTR (non-treatment related) deaths were excluded from analysis.

Treatment outcome was determined from tumor growth delay (TGD), which is defined as the increase in the median time to endpoint (TTE) in a treatment group compared to the control group:

$$\text{TGD} = \text{T} - \text{C},$$

expressed in days, or as a percentage of the median TTE of the control group:

$$\%\text{TGD} = \frac{\text{T} - \text{C}}{\text{C}} \times 100$$

where:

T = median TTE for a treatment group,

C = median TTE for the control group (Group 1)

Treatment may cause partial regression (PR) or complete regression (CR) of the tumor in an animal. In a PR response, the tumor volume is 50% or less of its Day 1 volume for three consecutive measurements during the course of the study, and equal to or greater than 13.5 $mm^3$ for one or more of these three measurements. In a CR response, the tumor volume is less than 13.5 $mm^3$ for three consecutive measurements during the course of the study. An animal with a CR response at the termination of a study is additionally classified as a long-term tumor-free survivor (LTTFS). Tumor regressions were monitored and recorded.

**SAMPLING**

In Groups 6-9, five mice per group were euthanized by terminal cardiac puncture under $CO_2$ anesthesia on Days 10, 13, 21 and 24, and gross necropsies were performed. Blood was collected into EDTA tubes, and complete blood counts (CBC) with differential were determined using a Cell-Dyn® 3700 System (Abbott Diagnostics) for automated hematology analyses. Reticulocyte values were determined. In addition, for Groups 1-5, five animals per group were euthanized at or just after endpoint by terminal cardiac puncture under $CO_2$ anesthesia, and gross necropsies were performed. Blood samples were collected into EDTA tubes, and CBC with differential and reticulocyte values were performed as previously described. All other animals in Groups 1-5 were euthanized at endpoint without sampling.

**TOXICITY**

Animals were weighed daily for the first five days of the study and then twice weekly. The mice were observed frequently for overt signs of any adverse, treatment-related side effects. Acceptable toxicity for cancer drugs in mice is defined by the NCI as a group mean body-weight loss of less than 20% during the test, and not more than one toxic death among ten treated animals.

**STATISTICAL AND GRAPHICAL ANALYSES**

The Logrank test was used to analyze the significance of the differences between the TTE values of treated and control groups. The Fisher's Exact test was employed to analyze the significance of differences in the number of 62-day survivors in Group 5 treated with the combination of carboplatin and the TPO mimetic peptide compound and Group 2 treated with carboplatin alone. For both tests, two-tailed statistical analyses were conducted at significance level P = 0.05, with results deemed significant at $0.01 \leq P \leq 0.05$, and highly significant at $P < 0.01$.

Median tumor growth curves show group median tumor volumes as a function of time. When an animal exited the study due to tumor size, the final tumor volume recorded for the animal was included with the data used to calculate the median volume at subsequent time points. Kaplan-Meier plots were constructed to show the percentage of animals remaining in the study as a function of time. These plots used the same data set as the Logrank test. Mean CBC values were plotted in bar graph form, with error bars showing one standard deviation of the mean. Prism (GraphPad) for Windows 3.03 was used for all graphic presentations and statistical analyses.

<u>RESULTS</u>

The individual times to endpoint by group for all animals in the study are shown in Figure 1. Figure 2 shows the median tumor growth curves (Figure 2a) and Kaplan-Meier plots (Figure 2b) for the groups in the study. Figures 3a - 3c show mean platelet, reticulocyte and hematocrit values, respectively, for Groups 6-9 on Days 10, 13, 21, and 24.

**EFFICACY**

***HT-29 Tumor Growth in Control Mice (Group 1)***

The tumors of all untreated Group 1 mice (n = 10) grew progressively to the 1000 $mm^3$ endpoint tumor volume with a median TTE of 24.8 days. Figure 1 shows the scatter plot of TTE values for this group, and the median tumor growth curve is included in Figure 2a.

***Response of HT-29 Xenografts to Carboplatin (Group 2)***

In Group 2 mice (n = 10) treated with carboplatin (60 mg/kg i.p. Days 1, 2, 12, 13), nine tumors grew to the endpoint tumor volume and one animal remained in the study on Day 62 with a tumor volume of 600 $mm^3$. No regression responses were recorded. The median TTE was 47.6 days, and corresponded to a statistically significant 22.8-day (92%) TGD (P = 0.001). The median tumor growth curve in Figure 2a illustrates the delay in tumor growth in Group 2 mice compared to untreated Group 1 controls.

**RESPONSE OF HT-29 XENOGRAFTS TO THE TPO MIMETIC PEPTIDE COMPOUND**

The tumors of all Group 3 mice (n = 10) treated with the TPO mimetic peptide compound (0.2 mg/kg i.v. Days 2, 13) grew progressively to the endpoint tumor volume. The Group 3 median TTE was 32.9 days and corresponded to a statistically non-significant 8.1-day (33%) TGD relative to Group 1 controls. The Group 3 median tumor growth curve

in Figure 2a is shifted slightly to the right of the curve of untreated Group 1 controls.

**Response of HT-29 Xenografts to the Combination of Carboplatin and the TPO mimetic peptide compound (Groups 4 and 5)**

The tumors of all Group 4 mice (n = 10) treated with the combination of carboplatin (60 mg/kg i.p. Days 1, 2, 12, 13) and two cycles of the TPO mimetic peptide compound (0.2 mg/kg i.v. Days 2, 13) grew progressively to the endpoint tumor volume. The median TTE was 54.2 days, and corresponded to a statistically significant 29.4-day (119%) TGD relative to untreated Group 1 controls ($P < 0.001$). However, TTE values in this group were not significantly different from those of Group 2 treated with carboplatin monotherapy. The Group 4 median tumor growth is very similar to the curve of Group 2 treated with carboplatin monotherapy (Figure 2a).

In Group 5 mice (n = 10) treated with the combination of carboplatin (60 mg/kg i.p. Days 1, 2, 12, 13) and three cycles of the TPO mimetic peptide compound (0.2 mg/kg i.v. Days 2, 13, 23), four tumors grew to the 1000 $mm^3$ endpoint tumor volume and six animals remained in the study on Day 62 with a median tumor volume of 877 $mm^3$ (see Table 4). No regression responses were recorded. The median TTE was 62.0 days and corresponded to a statistically significant 37.2-day (150%) TGD relative to untreated Group 1 controls ($P < 0.001$). When compared to Group 2 treated with carboplatin monotherapy, TGD in Group 5 approached statistical significance ($P = 0.067$). The increase in number of Group 5 62-day survivors (n = 6) also approached statistical significance when compared to the number of 62-day survivors (n = 1) in Group 2 carboplatin monotherapy-treated mice by Fisher's Exact analysis ($P = 0.057$). The median tumor growth curve for Group 5 is shifted slightly to the right of the curves for Groups 2 and 4. (Figure 2a)

**CBC ANALYSES**

Mean values for platelets (PLT), reticulocytes (RET), and hematocrit (HCT) for Groups 6-9 are presented graphically in Figures 3a - 3c.

**PLATELETS**

Figure 3a shows the mean platelet counts for Groups 6-9 on Days 10, 13, 21, and 23. Mean values were within the reference range established for female Harlan nude mice for all groups at all four time points. When compared to untreated Group 6 controls, carboplatin-treated mice (Group 7) had lower mean platelet counts at all time points and treatment with the TPO mimetic peptide compound (Group 8) resulted in higher mean platelet counts at all four time points.

Mice treated with the combination of carboplatin and the TPO mimetic peptide compound (Group 9) had mean platelet counts that were similar to those in the untreated control group. At each time point, mean platelet values in the combination treatment group (Group 9) were higher than those in Group 7 treated with carboplatin alone, with highest mean platelet count in Group 9 observed on Day 10.

**RETICULOCYTES**

Figure 3b shows mean reticulocyte values for Groups 6 - 9 on Days 10, 13, 21, and 23. Untreated controls (Group 6) and the TPO mimetic peptide compound-treated animals (Group 8) had mean reticulocyte values that remained relatively consistent at all time points, although mean values in Group 8 were slightly higher than in Group 6 controls. In carboplatin-treated animals (Group 7), mean reticulocyte values were negligible on Day 10 (near the expected nadir), higher than in control mice on Day 13, and then low on Day 21 after the second cycle of treatment. The combination treatment group (Group 9) followed a similar pattern, but the mean Day 10, 13 and 21 reticulocyte values in Group 9 were both higher than in Group 7 treated with carboplatin alone.

**HEMATOCRIT**

Figure 3c shows mean hematocrit (HCT) values for Groups 6 - 9 on Days 10, 13, 21, and 23. Mean HCT values were similar for untreated mice (Group 6) and the TPO mimetic peptide compound-treated mice (Group 8) at all time points. Animals treated with carboplatin only (Group 7) and the combination of carboplatin and the TPO mimetic peptide compound (Group 9) had lower mean HCT values than controls, with Group 7 mean HCT values being slightly lower than those of Group 9 on Days 13, 21, and 23.

**SIDE EFFECTS**

Animals were monitored for adverse treatment-related effects by frequent observation and body-weight (BW)

measurements (data not shown). The TPO mimetic peptide compound monotherapy (Group 3) was well tolerated, with no treatment-related (TR) deaths and no mean BW losses. Clinical observations were remarkable for large spleens in 2/5 necropsied Group 3 animals. The combination of the TPO mimetic peptide compound with carboplatin (Groups 4 and 5) resulted in similar BW mean losses compared to treatment with carboplatin alone (Group 2).

Clinical observations in Groups 2, 4 and 5 were remarkable for several mice with dark or mottled spleens upon necropsy. No TR mortality occurred in any group.

In summary, the TPO mimetic peptide compound did not enhance tumor growth. The addition of up to 3 cycles of the TPO mimetic peptide compound had no negative impact on tumor delay and in fact led to a small increase in survival when compared to carboplatin treatment alone.

**Human Studies**

The TPO mimetic peptide compound has been investigated in 2 human studies.

**Single Dose Study in Healthy Subjects**

Single i.v. doses of the TPO mimetic peptide compound up to and including 3 μg/kg were well-tolerated in healthy male subjects, with no apparent drug-related effects on adverse events, or cardiovascular or laboratory safety parameters (excluding platelet counts). Antibodies against the TPO mimetic peptide compound were not apparent in any post dose samples.

Single i.v. administration of the TPO mimetic peptide compound dose-dependently increased mean platelet count in healthy male subjects. Mean megakaryocyte ploidy was increased compared with placebo following doses of 1.5 μg/kg and above, and the mean number of CD62P+ platelets appeared to increase following administration of the 2 highest doses of the TPO mimetic peptide compound (although this was not reflected in other measures of platelet activation). Mean numbers of hematopoietic progenitor cells (burst-forming-unit erythroid [BFU-E] and peripheral CD34+ cells) increased compared with placebo following the highest dose of the TPO mimetic peptide compound.

The median $t_{max}$ of the TPO mimetic peptide compound ranged between 0.09 to 2 hours following single i.v. administration. In general, subjects showed more than 1 maximum in the profile, with a second maximum generally around 4 to 8 hours post dose. The mean terminal half-life was approximately 36 hours at 3 μg/kg: for most subjects there was limited data available in the terminal phase, therefore the half-life was not well defined. However, the elimination phase appeared consistent across the dose range. $C_{max}$ increased approximately dose proportionally. For AUC, no apparent dose proportionality could be determined across the 1.5- to 3-μg/kg dose range.

Example 2

**Multiple Dose Study in Cancer Subjects**

46 subjects with cancer receiving platinum-based therapies were enrolled into 3 cohorts (N = 16 in Cohort 1, 14 in Cohort 2, and 16 in Cohort 3). The subjects received the TPO mimetic peptide compound or placebo within 2 hours prior to the platinum-based chemotherapy on Day 1 of the first 2 cycles with a 21-day interval between each cycle. In addition to the Day 1 administration, gemcitabine administration was permitted on Day 8 of each cycle. Other chemotherapy medications were limited to dosing on Day 1 of each cycle only. Subjects were followed up for a total of 4 cycles of chemotherapy. Chemotherapy regimen continued beyond the first 4 cycles as per standard of care therapy. In the first cohort, 12 subjects received 1.5 μg/kg the TPO mimetic peptide compound and 4 subjects received placebo. In the second cohort 10 subjects received 3.0 μg/kg the TPO mimetic peptide compound and 4 subjects received placebo. In the third cohort, 12 subjects received 2.25 μg/kg the TPO mimetic peptide compound and 4 subjects received placebo. One of the dose escalation stopping criteria (platelet elevations of >3 times the baseline in 2 subjects) were met at 3.0 μg/kg dose. Therefore, a third cohort at a lower dose (2.25 μg/kg) was added to collect additional safety, tolerability, PD, and PK data. Preliminary safety, PK and PD data from these 3 cohorts at 1.5, 2.25, and 3.0 μg/kg are summarized below.

The distribution of tumor types and chemotherapy is detailed in Table 5.

**Table 5. Tumor Type and Chemotherapy**

| | **Placebo** | The TPO mimetic peptide compound | | |
|---|---|---|---|---|
| | **N = 12** | **1.5 μg/kg (N=12)** | **2.25 μg/kg (N=12)** | **3.0 μg/kg (N=10)** |
| **Tumor type (N)** | NSCLC (7)<br>SCLC (1)<br>Ovary (2)<br>Other (2) | Head and Neck (1)<br>NSCLC (1)<br>Ovary (3)<br>Pancreatic (2)<br>Cervix (1)<br>Bladder (1)<br>Other (3) | Gastric (2)<br>NSCLC (3)<br>Pancreatic (1)<br>SCLC (2)<br>Other (4) | NSCLC (2)<br>Ovary (4)<br>Cervix (1)<br>Other (2)<br>Colorectal (1) |
| **Platinum therapy (N): Mean dose (range)** | Carboplatin (8): 626 (430-909) mg<br>Cisplatin (4): 136 (116-148) | Carboplatin (6): 537 (379-800) mg<br>Cisplatin (6): 116 (50-140) mg | Carboplatin (9): 513 (339-700)<br>Cisplatin (3): 136 (100-168)<br>Oxaliplatin (1): 150 mg | Carboplatin (7): 516 (300-730) mg<br>Cisplatin (2): 103 (80-125) mg<br>Oxaliplatin (1): 220 mg |
| **Chemotherapy (N): Mean dose (range)** | Gemcitabine (8): 2002 (1200-2339)<br><br>Paclitaxel (4): 325 (290-400) | Gemcitabine (10): 1624 (497-2044) mg<br><br>Paclitaxel (1): 277 mg<br>5-FU (1): 5782 mg | Gemcitabine (4): 1888 (1520-2625)<br><br>Epirubicin (1): 100 mg<br>Paclitaxel (6): 286 (243-324)<br>Irinotecan (1): 270 mg | Gemcitabine (5): 1577 (1000-1980) mg<br>Paclitaxel (4): 294 (270-330) mg |

Pharmacokinetics at 1.5 μg/kg dose was similar to those in healthy subjects. Placebo subjects from all cohorts were pooled.

At the 1.5 μg/kg dose there was no apparent difference in the platelets as compared to the placebo subjects. However, at the 2.25 and 3 μg/kg dose, platelet nadir and peak platelet counts were approximately 2-fold higher relative to placebo (Figure 4, Table 6 and 7). Mean platelet nadir was observed on Day 10 for the 2.25 and 3 μg/kg dose groups, but for the placebo and 1.5 μg/kg dose, platelets continued to decline up to Day 15. Mean peak platelets were observed on Day 15 for the 3.0 μg/kg dose but for the placebo, 2.25 and 1.5 μg/kg doses, peak platelets were observed on Day 21.

Two subjects at the 3.0 μg/kg dose had a transient platelet increase of more than 3 times the baseline in the first cycle (stopping criteria for further dose escalation) but no subjects in the 1.5 and 2.25 μg/kg dose group met the stopping criteria.

The platelet elevations were attenuated in the second cycle and remained below 3 times the baseline in all subjects. These results at the 2.25 and 3 μg/kg dose, indicate a reduction in chemotherapy-induced decline in platelets and faster recovery relative to placebo, suggesting potential for the TPO mimetic peptide compound in prevention of CIT (Figure 4, Table 6 and 7).

A total of 7 subjects were excluded from the preliminary statistical analysis of Day 42 platelet count data and 9 subjects were excluded from the preliminary statistical analysis of Day 42 hemoglobin data due to either incorrect randomization, platelet transfusion, not administered the 2$^{nd}$ dose of study medication, or lost to follow-up.

**Table 6:** Minimum Platelet Counts

| Cycle | Treatment Group | N | Geometric Mean | GMR (Active/Placebo) | 95% Confidence Interval |
|---|---|---|---|---|---|
| Cycle 1 | Placebo | 12 | 94.13 | | |
| | 1.5 μg/kg | 9 | 47.01 | 0.5 | (0.2, 1.2) |
| | 2.25 μg/kg | 11 | 213.46 | 2.3 | (1.0, 5.2) |
| | 3.0 μg/kg | 9 | 191.17 | 2.0 | (0.8, 5.1) |
| Cycle 2 | Placebo | 12 | 78.67 | | |

(continued)

| Cycle | Treatment Group | N | Geometric Mean | GMR (Active/Placebo) | 95% Confidence Interval |
|---|---|---|---|---|---|
| | 1.5 μg/kg | 8 | 75.10 | 1.0 | (0.6, 1.6) |
| | 2.25 μg/kg | 10 | 175.08 | 2.2 | (1.4, 3.7) |
| | 3.0 μg/kg | 9 | 170.16 | 2.2 | (1.3, 3.7) |
| Across | Placebo | 12 | 60.96 | | |
| | 1.5 μg/kg | 9 | 33.99 | 0.6 | (0.3, 1.2) |
| | 2.25 μg/kg | 11 | 163.71 | 2.7 | (1.3, 5.4) |
| | 3.0 μg/kg | 9 | 121.61 | 2.0 | (0.9, 4.3) |

**Table 7:** Maximum Platelet Counts

| Cycle | Treatment Group | N | Geometric Mean | GMR (Active/Placebo) | 95% Confidence Interval |
|---|---|---|---|---|---|
| Cycle 1 | Placebo | 12 | 301.84 | | |
| | 1.5 μg/kg | 9 | 331.25 | 1.1 | (0.8, 1.5) |
| | 2.25 μg/kg | 11 | 557.78 | 1.8 | (1.4, 2.5) |
| | 3.0 μg/kg | 9 | 641.56 | 2.1 | (1.5, 3.0) |
| Cycle 2 | Placebo | 12 | 289.10 | | |
| | 1.5 μg/kg | 8 | 290.55 | 1.0 | (0.7, 1.5) |
| | 2.25 μg/kg | 10 | 397.37 | 1.4 | (0.9, 2.0) |
| | 3.0μg/kg | 9 | 401.20 | 1.4 | (0.9, 2.1) |
| Across | Placebo | 12 | 356.50 | | |
| | 1.5 μg/kg | 9 | 371.53 | 1.0 | (0.8, 1.4) |
| | 2.25 μg/kg | 11 | 570.39 | 1.6 | (1.2, 2.1) |
| | 3.0 μg/kg | 9 | 633.09 | 1.8 | (1.3, 2.4) |

Preliminary evaluation of the change in hemoglobin levels from baseline to the end of Cycle 2 (Day 42) and beyond also suggests a dose-related trend for preservation of hemoglobin (Figure 5, Table 8).

**Table 8:** Statistical Analysis of the TPO mimetic peptide compound Change From Baseline in Hemoglobin at Days 42 63 and 84

| Day | Treatment Group | N | LS Mean (SE) | Reference Group | Diff of LS Mean (SE) | 95% Confidence Interval |
|---|---|---|---|---|---|---|
| Day 42 | Placebo | 12 | -2.17 (0.39) | | | |
| | 1.5 μg/kg | 7 | -1.63 (0.50) | Placebo | 0.54 (0.63) | (-0.8, 1.8) |
| | 2.25 μg/kg | 10 | -1.60 (0.42) | Placebo | 0.57 (0.57) | (-0.6, 1.7) |
| | 3.0 μg/kg | 8 | -1.16 (0.47) | Placebo | 1.00 (0.61) | (-0.2, 2.2) |
| Day 63 | Placebo | 9 | -2.23 (0.57) | | | |
| | 1.5 μg/kg | 6 | -2.98 (0.70) | Placebo | -0.75 (0.90) | (-2.6, 1.1) |
| | 2.25 μg/kg | 8 | -1.55 (0.61) | Placebo | 0.68 (0.83) | (-1.0, 2.4) |
| | 3.0 μg/kg | 8 | -1.44 (0.61) | Placebo | 0.79 (0.83) | (-0.9, 2.5) |
| Day 84 | Placebo | 8 | -1.93 (0.48) | | | |
| | 1.5 μg/kg | 6 | -1.88 (0.55) | Placebo | 0.05 (0.73) | (-1.5, 1.6) |
| | 2.25 μg/kg | 5 | -1.56 (0.61) | Placebo | 0.37 (0.77) | (-1.2, 2.0) |
| | 3.0 μg/kg | 5 | -0.36 (0.61) | Placebo | 1.57 (0.77) | (-0.0, 3.2) |

Although adverse events were observed (see Table 9), the adverse events did not appear to be related to increasing doses of the TPO mimetic peptide compound; did not appear to be different from placebo group; and appeared to be those commonly reported with the chemotherapy utilized in the study.

**Table 9.** Treatment-Emergent Adverse Events by Body System and Preferred Term

| **Body System or Organ Class** Dictionary-derived Term | Placebo (N=12) N | 1.5 ug/kg (N=12) n | 2.25 ug/kg (N=12) n | 3.0 ug/kg (N=10) n |
|---|---|---|---|---|
| **Total no. subjects WITH ADVERSE EVENTS** | 11 | 12 | 10 | 9 |
| **Gastrointestinal disorders** | 9 | 7 | 7 | 6 |
| Nausea | 8 | 6 | 2 | 2 |
| Vomiting | 8 | 2 | 6 | 2 |
| Constipation | 3 | 3 | 2 | 4 |
| Abdominal pain | 0 | 2 | 1 | 2 |
| Diarrhea | 1 | 2 | 1 | 1 |
| Dyspepsia | 2 | 0 | 0 | 0 |
| Stomatitis | 0 | 2 | 0 | 0 |
| Eructation | 0 | 0 | 1 | 0 |
| Hemorrhoids | 0 | 1 | 0 | 0 |
| **Blood and lymphatic system disorders** | 8 | 8 | 6 | 3 |
| Neutropenia | 6 | 3 | 4 | 3 |
| Anemia | 1 | 7 | 2 | 1 |
| Leukopenia | 3 | 2 | 3 | 2 |
| Thrombocytopenia | 5 | 3 | 0 | 2 |
| Lymphopenia | 0 | 2 | 0 | 0 |
| Febrile neutropenia | 0 | 0 | 1 | 0 |
| Leukocytosis | 0 | 0 | 0 | 1 |
| Thrombocythaemia | 0 | 1 | 0 | 0 |
| **General disorders and administration site conditions** | 7 | 7 | 3 | 6 |
| Fatigue | 4 | 5 | 1 | 4 |
| Pyrexia | 2 | 1 | 3 | 2 |
| Pain | 0 | 3 | 0 | 0 |
| Oedema | 0 | 0 | 1 | 1 |
| Chills | 0 | 0 | 1 | 0 |
| General physical health deterioration | 0 | 0 | 1 | 0 |
| Mucosal inflammation | 1 | 0 | 0 | 0 |
| Oedema peripheral | 0 | 1 | 0 | 0 |
| **Metabolism and nutrition disorders** | 5 | 5 | 2 | 3 |
| Anorexia | 5 | 5 | 0 | 2 |
| Dehydration | 0 | 0 | 1 | 1 |
| Hyponatraemia | 0 | 0 | 2 | 0 |
| Diabetes mellitus non-insulin-dependent | 0 | 1 | 0 | 0 |
| Hypoalbuminaemia | 0 | 0 | 1 | 0 |
| Hypocalcaemia | 0 | 1 | 0 | 0 |
| Hypokalaemia | 0 | 0 | 1 | 0 |
| Hypomagnesaemia | 0 | 0 | 1 | 0 |
| Hypophosphataemia | 0 | 1 | 0 | 0 |

(continued)

| **Body System or Organ Class** Dictionary-derived Term | Placebo (N=12) N | 1.5 ug/kg (N=12) n | 2.25 ug/kg (N=12) n | 3.0 ug/kg (N=10) n |
|---|---|---|---|---|
| **Skin and subcutaneous tissue disorders** | 4 | 4 | 3 | 3 |
| Alopecia | 2 | 2 | 3 | 2 |
| Rash | 1 | | 0 | 0 |
| Dermatitis acneiform | 1 | 0 | 0 | 0 |
| Dry skin | 0 | 0 | 0 | 1 |
| **Nervous system disorders** | 4 | 3 | 3 | 2 |
| Headache | 1 | | 1 | 1 |
| Dizziness | 1 | 0 | 2 | 0 |
| Paraesthesia | 2 | 0 | 0 | 1 |
| Peripheral motor neuropathy | 1 | | 0 | 0 |
| Neuropathy peripheral | 0 | 1 | 0 | 0 |
| Peripheral sensory neuropathy | 1 | 0 | 0 | 0 |
| **Investigations** | 1 | | 2 | 4 |
| Blood creatinine increased | 0 | 1 | 1 | 1 |
| Haematocrit decreased | 0 | 0 | 1 | 1 |
| Weight decreased | 1 | | 0 | 0 |
| Alanine aminotransferase increased | 0 | 0 | 0 | 1 |
| Aspartate aminotransferase increased | 0 | 0 | 0 | 1 |
| C-reactive protein increased | 0 | 0 | 0 | 1 |
| Platelet count decreased | 0 | 1 | 0 | 0 |
| **Respiratory, thoracic and mediastinal disorders** | 2 | 2 | 3 | 1 |
| Cough | 1 | 0 | 2 | 0 |
| Dyspnoea | 0 | 1 | 1 | 0 |
| Epistaxis | 1 | 0 | 0 | 1 |
| Hiccups | 0 | 1 | 1 | 0 |
| Respiratory failure | 0 | 1 | 0 | 0 |
| **Infections and infestations** | 1 | 4 | 0 | 0 |
| Infection | 0 | 2 | 0 | 0 |
| Folliculitis | 0 | 1 | 0 | 0 |
| Gastrointestinal infection | 1 | 0 | 0 | 0 |
| Lung abscess | 0 | 1 | 0 | 0 |
| Respiratory tract infection | 0 | 1 | 0 | 0 |
| **Musculoskeletal and connective tissue disorders** | 1 | 0 | 1 | 2 |
| Arthralgia | 1 | 0 | 0 | 1 |
| Pain in extremity | 0 | 0 | 1 | 1 |

| **Body System or Organ Class** Dictionary-derived Term | Placebo (N=12) N | 1.5 ug/kg (N=12) n | 2.25 ug/kg (N=12) n | 3.0 ug/kg (N=10) n |
|---|---|---|---|---|
| **Psychiatric disorders** | 0 | 1 | 2 | 1 |
| Insomnia | 0 | 0 | 1 | 1 |
| Anxiety | 0 | 1 | 0 | 0 |

(continued)

| Body System or Organ Class<br>Dictionary-derived Term | Placebo (N=12)<br>N | 1.5 ug/kg (N=12)<br>n | 2.25 ug/kg (N=12)<br>n | 3.0 ug/kg (N=10)<br>n |
|---|---|---|---|---|
| **Psychiatric disorders** | 0 | 1 | 2 | 1 |
| Confusional state | 0 | 0 | 1 | 0 |
| **Cardiac disorders** | 0 | 3 | 0 | 0 |
| Tachycardia | 0 | 2 | 0 | 0 |
| Acute myocardial infarction | 0 | 1 | 0 | 0 |
| Atrial fibrillation | 0 | 1 | 0 | 0 |
| Cardiac failure | 0 | 1 | 0 | 0 |
| **Ear and labyrinth disorders** | 1 | 0 | 0 | 1 |
| Tinnitus | 1 | 0 | 0 | 1 |
| **Vascular disorders** | 0 | 0 | 2 | 0 |
| Hypertension | 0 | 0 | 2 | 0 |
| **Hepatobiliary disorders** | 0 | 0 | 1 | 0 |
| Hyperbilirubinaemia | 0 | 0 | 1 | 0 |
| **Immune system disorders** | 0 | 0 | 0 | 1 |
| Hypersensitivity | 0 | 0 | 0 | 1 |
| **Neoplasms benign, malignant and unspecified (incl cysts and polyps)** | 0 | 1 | 0 | 0 |
| Cancer pain | 0 | 1 | 0 | 0 |
| **Renal and urinary disorders** | 0 | 0 | 1 | 0 |
| Azotaemia | 0 | 0 | 1 | 0 |

Serious adverse events for each treatment group are summarized in Table 10. There were 21 serious adverse events (SAEs) reported by 9 subjects. With exception of one case of thrombocythemia, all other SAE's were classified as unrelated to the TPO mimetic peptide compound. Two of the 21 SAEs reported resulted in death, one subject had cardiac failure and another subject had acute myocardial infarction. The thrombocythemia observed in a subject was reported as being 'very likely' related to the TPO mimetic peptide compound. This subject had concurrent severe lung infection and platelets above the normal range at baseline. The subject had thoracotomy and was prescribed antibiotics. This subject also had chronically elevated platelets. Chronically elevated platelets are observed in some subjects with lung cancer.

**Table 10:** Treatment-Emergent Serious Adverse Events

| Body System or Organ Class<br>Dictionary-derived Term | Placebo (N=12)<br>n | 1.5 ug/kg (N=12)<br>n | 2.25 μg/kg (N=12)<br>n | 3.0 ug/kg (N=10)<br>n |
|---|---|---|---|---|
| **Total no. subjects WITH SERIOUS ADVERSE EVENTS** | 1 | 5 | 3 | 0 |
| **Blood and lymphatic system disorders** | 1 | 2 | 1 | 0 |
| Febrile neutropenia | 0 | 0 | 1 | 0 |
| Neutropenia | 0 | 1 | 0 | 0 |

(continued)

| Body System or Organ Class<br>Dictionary-derived Term | Placebo (N=12)<br>n | 1.5 ug/kg (N=12)<br>n | 2.25 μg/kg (N=12)<br>n | 3.0 ug/kg (N=10)<br>n |
|---|---|---|---|---|
| **Total no. subjects WITH SERIOUS ADVERSE EVENTS** | 1 | 5 | 3 | 0 |
| **Blood and lymphatic system disorders** | 1 | 2 | 1 | 0 |
| Thrombocythaemia | 0 | 1 | 0 | 0 |
| Thrombocytopenia | 1 | 0 | 0 | 0 |
| **Gastrointestinal disorders** | 0 | 2 | 1 | 0 |
| Abdominal pain | 0 | 1 | 0 | 0 |
| Nausea | 0 | 1 | 0 | 0 |
| Vomiting | 0 | 0 | 1 | 0 |
| **Metabolism and nutrition disorders** | 0 | 1 | 2 | 0 |
| Hyponatraemia | 0 | 0 | 2 | 0 |
| Anorexia | 0 | 1 | 0 | 0 |
| Dehydration | 0 | 0 | 1 | 0 |
| **Cardiac disorders** | 0 | 2 | 0 | 0 |
| Acute myocardial infarction | 0 | 1 | 0 | 0 |
| Atrial fibrillation | 0 | 1 | 0 | 0 |
| Cardiac failure | 0 | 1 | 0 | 0 |
| **General disorders and administration site conditions** | 0 | 2 | 0 | 0 |
| Fatigue | 0 | 1 | 0 | 0 |
| Pain | 0 | 1 | 0 | 0 |
| Pyrexia | 0 | 1 | 0 | 0 |
| **Infections and infestations** | 0 | 2 | 0 | 0 |
| Lung abscess | 0 | 1 | 0 | 0 |
| Respiratory tract infection | 0 | 1 | 0 | 0 |
| **Respiratory, thoracic and mediastinal disorders** | 0 | 1 | 1 | 0 |
| Dyspnoea | 0 | 0 | 1 | 0 |
| Respiratory failure | 0 | 1 | 0 | 0 |
| **Investigations** | 0 | 0 | 1 | 0 |
| Blood creatinine increased | 0 | 0 | 1 | 0 |

Example 3

Lung cancer is the leading cause of cancer deaths in the US, with 213,380 new cases and 160,390 deaths in 2007, and non-small cell histology accounts for 80-85% of all cases.[24] At initial staging, approximately 32% of subjects are found to have Stage III disease and 36% have stage IV disease, with five-year survival rates of 8.4% and 1.6%, respectively.[25]

For subjects with Stage IIIB or IV disease, doublet chemotherapy remains the standard, with a platinum-analog as part of the regimen, and often additional radiation for Stage IIIB subjects. Cisplatin or carboplatin have been most commonly tested in combination with other agents, with results for doublet therapy producing modest survival benefits overall.[4, 26-36] A key study, ECOG 1594, compared four different regimens and demonstrated a longer median time to progression for the combination of gemcitabine and cisplatin (4.5 months, 95% CI 3.7-4.8, p=0.001) compared to the other three doublets; but there was no overall survival advantage.4 The median overall survival for gemcitabine-containing

regimens is nine months, and 1-year survival has ranged from 32-40% in various studies.[28,33-35,37] Progression-free survival (PFS) at one year was 14% in one study.[34] In these studies, subjects with performance status 2 did not account for more than 10-15% of those enrolled; the remainder had performance status 0-1.

Recently, the addition of bevacizumab to paclitaxel/carboplatin has been shown to extend overall survival for subjects with Stage IIIB/IV NSCLC,[38] and has also been evaluated in combination with cisplatin/gemcitabine with demonstration of an improvement in progression-free survival, but not yet overall survival.[39] Toxicities with bevacizumab have somewhat limited its use, however, to selected NSCLC populations.[40]

The toxicity of gemcitabine-containing doublet regimens has been significant in many cases, with hematological toxicity figuring prominently in the safety profile. Grade 3-4 thrombocytopenia has been reported to range from 24 to 56%, and grade 3-4 anemia has been reported in up to 28% of subjects who have received regimens containing either cisplatin/gemcitabine or carboplatin/gemcitabine.[28,33-35,41,42] Among studies where transfusion rates have been described, RBC transfusions and platelet transfusions have been reported in 37 to 41% and 6 to 20% of subjects who were treated with gemcitabine/platinum-analog doublets, respectively.[33,34,35] Information on the use of erythropoiesis-stimulating agents was not reported. Hematological toxicities have often led to dose reductions or dosing delays, so the rationale exists for attempting to deliver more complete dosing if significant chemotherapy-induced anemia and/or thrombocytopenia can be mitigated. Thus, it is reasonable to evaluate the potential for the TPO mimetic peptide compound to prevent or reduce anemia or thrombocytopenia in subjects who are receiving either gemcitabine/cisplatin or gemcitabine/carboplatin chemotherapy for advanced NSCLC.

**Overall Rationale for the Study**

Platinum chemotherapy agents (e.g., carboplatin, cisplatin) and platinum-based chemotherapy regimens (e.g., carboplatin and cisplatin given alone or in combination with gemcitabine) are used for the treatment of different types of cancers and have been shown to cause clinically significant bone marrow suppression, leading to decreases in WBCs, RBCs, and platelets, resulting in neutropenia, anemia, and thrombocytopenia, respectively.

The consequences of myelosuppressive chemotherapy regimens include anemia and/or thrombocytopenia, which can result in impairment in daily activities due to fatigue, the need for RBC or platelet transfusions or treatment with ESAs, delays in chemotherapy schedule, chemotherapy dose reductions, and possibly decreased survival.

Preclinical pharmacology studies of the TPO mimetic peptide compound have demonstrated a myeloprotective effect of the TPO mimetic peptide compound in prevention of carboplatin or carboplatin and gemcitabine-induced anemia and thrombocytopenia.[23]

Data from studies conducted in healthy subjects and cancer subjects receiving platinum-based chemotherapy are consistent with the efficacy, safety, PK, and PD findings in preclinical studies. These findings suggest that the TPO mimetic peptide compound should be efficacious in the prevention of CIA and/or CIT in cancer subjects receiving platinum-based chemotherapy.

**Objectives**

To evaluate the efficacy of the TPO mimetic peptide compound on the prevention of CIA in subjects with non-small cell lung cancer (NSCLC) receiving a 21-day chemotherapy regimen of gemcitabine and either carboplatin or cisplatin.

To evaluate the efficacy of the TPO mimetic peptide compound on the prevention of CIT in subjects with NSCLC receiving a 21-day chemotherapy regimen of gemcitabine and either carboplatin or cisplatin.

To evaluate the safety, pharmacokinetics (PK), and pharmacodynamics (PD) of the TPO mimetic peptide compound in subjects with NSCLC receiving a 21-day chemotherapy regimen of gemcitabine and either carboplatin or cisplatin.

To evaluate the effect of the TPO mimetic peptide compound on subject-reported outcome (PRO) assessments, and to further validate these assessments, in subjects with NSCLC receiving a 21-day chemotherapy regimen of gemcitabine and either carboplatin or cisplatin.

**Hypotheses**

In subjects with NSCLC receiving a 21-day chemotherapy regimen of gemcitabine and either carboplatin or cisplatin, co-administration of the TPO mimetic peptide compound provides a lower incidence rate of the composite endpoint of Grade 2 or higher anemia, or a $\geq$ 2 g/dL drop in hemoglobin on the first day of any chemotherapy cycle (Cycle 2 to 6) relative to baseline (Cycle 1, Day 1), or the use of rescue intervention for anemia (e.g., erythropoiesis stimulating agents [ESAs], red blood cell [RBC] transfusion) as compared to placebo.

In subjects with NSCLC receiving a 21-day chemotherapy regimen of gemcitabine and either carboplatin or

cisplatin, co-administration of the TPO mimetic peptide compound provides a lower incidence rate of the composite endpoint of Grade 2 or higher thrombocytopenia or the use of platelet transfusion as compared to placebo.

**OVERVIEW OF STUDY DESIGN**

In this study, the dose of the TPO mimetic peptide compound or placebo (i.e., dosing solution volume) for each subject is planned to be fixed in Cycles 1 and 2, then modified in subsequent cycles, if necessary, based on the Day 15 platelet count of the previous chemotherapy cycle to optimize subject safety. To further maximize subject safety, hemoglobin values for each subject will also be evaluated on Day 1 of each chemotherapy cycle to determine if the dose of study medication should be held or if a subject should be discontinued from the study. The dose of the TPO mimetic peptide compound or placebo will not be modified based on hemoglobin values.

Study Design

Subjects with stage IIIB or IV NSCLC, eligible to receive up to 6 cycles of a 21-day chemotherapy regimen of gemcitabine and either carboplatin or cisplatin, will be enrolled prior to their first chemotherapy cycle.

For each subject, the study will consist of approximately 24 visits. Subject visits will be at: Screening (Visit 1; within Day -14 to Day -1); Day 1, 8, and 15 of Cycle 1 to 6 (Visit 2 to 19); and 5 follow up visits at 30 days after the last dose administration of study medication (Visit 20), and then 6 months (Visit 21), 12 months (Visit 22), 18 months (Visit 23), and 24 months (Visit 24) after Day 1 of Cycle 1 (i.e., 1st dose of chemotherapy). Visit 22, 23, and 24 will require only a telephone call and not a visit to the investigative center. The study duration for each subject will be approximately 24 months (Screening through final follow up visit).

Subjects who meet the entry criteria will be randomly assigned to receive the TPO mimetic peptide compound (n = 74) or placebo (n= 74). The study medication will be administered as an IV bolus on Day 1 of each chemotherapy cycle, within 2 hours prior to receiving chemotherapy.

In a previous study, the maximal effect of the TPO mimetic peptide compound on platelet count was observed on Day 15. Therefore, to ensure subject safety, the dose of the TPO mimetic peptide compound in Cycle 2 to 6 will be adjusted, if necessary, based on the subject's Day 15 platelet count in the previous cycle.

An overview of the planned the TPO mimetic peptide compound and placebo doses for each cycle are described in Table 11.

**Table 11.** Overview of the TPO mimetic peptide compound and Placebo Doses for Cycle 1 to 6

| **Cycle** | The TPO mimetic peptide compound Dose (μg/kg) |
|---|---|
| 1 | 2.5 |
| 2 | 3.0 [1] |
| 3 | 2.0 to 3.5 [2] |
| 4 | 2.0 to 3.5 [2] |
| 5 | 2.0 to 3.5 [2] |
| 6 | 2.0 to 3.5 [2] |

[1] If necessary, the dose will be reduced to 2.5 μg/kg or withheld based on the subject's platelet count on Cycle 1, Day 15 and Cycle 2, Day 1.
[2] If necessary, the dose will be titrated or withheld based on the subject's platelet count on Day 15 of the preceding Cycle and Day 1 of this Cycle.

**On Day 1 in Cycle 2,** each subject will receive 3.0 μg/kg of the TPO mimetic peptide compound or placebo. In the event a subject's Cycle 1 Day 15 platelet count is >700,000/μL, and the platelet count remains >500,000/μL but is < 700,000/μL on Day 1 of Cycle 2, the subject will receive 2.5 μg/kg of the TPO mimetic peptide compound or placebo. If the platelet count is > 700,000/μL on Day 1 of Cycle 2, the subject will not be dosed with the TPO mimetic peptide compound or placebo in Cycle 2.

**On Day 1 of Cycle 3 to 6,** the dose of the TPO mimetic peptide compound or placebo (i.e., dosing solution volume) will be based on the subject's Day 15 platelet count in the previous cycle.

In Cycle 2 to 5, if a subject's Day 15 platelet count is > 700,000/μL, and the platelet count on Day 1 of the next chemotherapy cycle remains > 700,000/μL, the subject will not be dosed with the TPO mimetic peptide compound or

placebo for that given cycle. Subjects could be dosed again for subsequent cycles if platelets are < 700,000/μL on Day 1 of that chemotherapy cycle.

In order to further maximize subject safety, hemoglobin values for each subject will also be evaluated on Day 1 of each chemotherapy cycle to determine if the dose of the TPO mimetic peptide compound or placebo should be held. Specifically, if a subject has a hemoglobin value >15 g/dL or has an increase from baseline of ≥ 2 g/dL on Day 1 of any cycle, the subject will not be dosed with the TPO mimetic peptide compound for that given cycle. The dose of the TPO mimetic peptide compound or placebo will not be modified based on hemoglobin values.

A detailed dose titration scheme is provided in Table 12.

**Table 12.** The TPO mimetic peptide compound Dose Titration Scheme for Cycle 3 to 6

| Day 15 platelet count of previous chemotherapy cycle (Cycle 2 to 5) x 1000 (/μL) | The TPO mimetic peptide compound dose (μg/kg) [2,3] for next chemotherapy cycle (Cycle 3 to 6) |
|---|---|
| ≥ 900 [1] | 2.0 |
| 501 - 899 [1] | 2.5 |
| 101 - 500 | 3.0 |
| 50 - 100 | 3.25 |
| < 50 | 3.5 |

[1] If the subject's platelet count on Day 1 of the next chemotherapy cycle is > 700,000/μL, the subject will not be dosed with the TPO mimetic peptide compound or placebo for that given cycle. Subjects could be dosed again for subsequent cycles if platelets are < 700,000/μL on Day 1 of that chemotherapy cycle. If platelet count continues to be > 700,000/μL on Day 1 of two consecutive cycles, the subject will be discontinued from the study.

[2] Placebo subjects will receive the same dosing solution volume of the corresponding THE TPO PEPTIDE COMPOUND dose.

[3] If a subject has a hemoglobin value >15 g/dL or has an increase from baseline of ≥ 2 g/dL on Day 1 of any cycle, the subject will not be dosed with the TPO peptide compound for that given cycle. If hemoglobin continues to be above >15 g/dL, or there is ≥ 2 g/dL increase from baseline on Day 1 of two consecutive cycles, the subject will be discontinued from the studv.

The control treatment arm (placebo) will be used to establish the frequency and/or magnitude of changes in laboratory and/or clinical endpoints and adverse events that may occur with chemotherapy and standard of care therapies in the absence of the TPO mimetic peptide compound treatment.

This study is designed to assess the efficacy, safety, PK, and PD of the TPO mimetic peptide compound in male and female subjects with Stage IIIB or IV NSCLC receiving a combination chemotherapy regimen of gemcitabine and either carboplatin or cisplatin.

The NSCLC population was chosen because the incidence of anemia is high, with Grade 2 or higher anemia reported to range from 38 to 71 % with platinum analogs and gemcitabine combinations. The incidence of Grade 3 or higher anemia in this setting has been reported to be as high as 28% (range 5 to 28%), and the incidence of transfusion in this setting has been reported to be approximately 39%. [3,4]

At the time of diagnosis, approximately 68% of the subjects are found to have stage IIIB/IV NSCLC. The 5-year survival is poor (8.4% for stage IIIB and 1.6% for stage IV), in addition, the median overall survival for stage IIIB/IV NSCLC subjects receiving gemcitabine-containing regimens is 9 months. The limited median survival time for these subjects will also permit an exploratory assessment of the safety of the TPO mimetic peptide compound with respect to tumor progression and overall survival in a relatively short period.

All subjects will receive standard of care treatments, including rescue interventions if necessary, so that clinical outcome for each subject with respect to standard of care treatment is optimized. Subjects will be randomized to receive the TPO mimetic peptide compound or placebo in addition to their standard of care treatment for NSCLC. Erythropoietin, along with other non-investigational hematopoietic agents such as iron, vitamin B, folic acid and red blood cell transfusions, will be allowed as standard of care medicines to treat anemia during the cycles of chemotherapy. The placebo treatment will be used to establish the frequency and magnitude of changes in laboratory and/or clinical endpoints that may occur in the absence of the TPO mimetic peptide compound treatment and thus the placebo dose cohort will allow establishment of the safety of the TPO mimetic peptide compound treatment.

The composite endpoint of anemia will include the incidence of (1) Grade 2 or higher anemia (i.e. Hb of < 10 g/dL), or (2) a ≥ 2 g/dL drop in hemoglobin on the first day of any chemotherapy cycle (Cycle 2 to 6) relative to baseline (Cycle 1, Day 1), or (3) use of rescue intervention for anemia (e.g., RBC transfusion, ESAs). The components of the

composite endpoint were selected based on the following: the current treatment paradigm for ESA use (i.e. ESA use is permitted when the subject's hemoglobin approaches 10 g/dL or is <10 g/dL), a ≥ 2 g/dL drop in Hb from baseline is considered clinically meaningful, and ESAs and RBC transfusions are rescue interventions for anemia that are accepted as clinically significant events.

The required chemotherapy regimen of gemcitabine and either carboplatin or cisplatin every 21 days was selected because it is an effective regimen for the treatment of NSCLC. Cancer subjects receiving platinum-based therapies have been reported to demonstrate a decrease in hemoglobin of about 0.5 g/dL at each chemotherapy cycle. The median number of treatment cycles in these subjects has been reported to be 4. Therefore, a decrease in hemoglobin of approximately 2 to 3 g/dL from baseline in the non-TPO mimetic peptide compound treated arm over the 4-6 treatment cycles is anticipated.

The composite endpoint of thrombocytopenia will include the incidence of (1) Grade 2 or higher thrombocytopenia or (2) the use of platelet transfusion. The components of the composite endpoint were selected based on the following: Grade 2 or higher thrombocytopenia (< 75,000 /μL platelet count) has been associated with delays in surgical procedures and chemotherapy treatments due to the potential for an increased risk of bleeding, and the use of platelet transfusion is a clinically significant event that is performed to stop or prevent bleeding due to thrombocytopenia.

Randomization will be stratified based on platinum-based chemotherapy (i.e., carboplatin or cisplatin) and stage of disease (i.e., Stage IIIB or IV) to maintain balance in the active and placebo groups. Carboplatin and cisplatin have similar efficacy profiles and are used with gemcitabine extensively, depending up on the preferences at each treatment center. Randomization will be stratified based on the platinum chemotherapy agent due to their different toxicity profiles. Carboplatin is less nephrotoxic and less emetogenic than cisplatin, and neurotoxicity and ototoxicity are virtually absent. Myelosuppression is the major toxic effect of carboplatin. In contrast, the major toxicities for cisplatin have been nausea, vomiting, and generalized gastrointestinal effects including post-platinum diarrhea. Hydration and dose fractionation mitigate most of the nephrotoxic effect of the cisplatin. The neuropathic effects for cisplatin are relatively common and are related to the cumulative dose administered. Although differences in the safety and efficacy of the TPO mimetic peptide compound in Stage IIIb and IV cancer subjects are not anticipated, the randomization will be stratified by the stage of the disease to detect any potential differences.

This study is designed as an adaptive dose trial to maximize the safety of each subject by minimizing a transient increase in platelet counts above the normal range.

The TPO mimetic peptide compound or placebo will be administered within 2 hours prior to chemotherapy. This dose timing is based on the preclinical findings, which indicate that the TPO mimetic peptide compound needs to be administered within one day of the chemotherapy. Dosing within 2 hours prior to chemotherapy in subjects has been chosen with respect to practical considerations of giving chemotherapy supportive care agents (e. g. antiemetics to minimize nausea and vomiting, hydration to minimize nephrotoxiciy) prior to chemotherapy as well as minimize the variability in response to the TPO mimetic peptide compound. Each subject will receive an intravenous bolus dose of the TPO mimetic peptide compound or placebo on Day 1 of each chemotherapy cycle starting from the first cycle up to 6 cycles.

In Cycle 1 and 2, fixed doses of the TPO mimetic peptide compound or placebo (i.e., dosing solution volume) will be administered. In cycle 1, 2.5 μg/kg dose of the TPO mimetic peptide compound or placebo will be administered. This dose has been chosen based on the results from study in Example 2, where fixed doses ranging from 1.5 - 3.0 μg/kg were administered. On Day 1 in Cycle 2, each subject will receive 3.0 μg/kg of the TPO mimetic peptide compound or placebo. In the event a subject's Cycle 1 Day 15 platelet count is >700,000/μL, and the platelet count remains >500,000/μL but is < 700,000/μL on Day 1 of Cycle 2, the subject will receive 2.5 μg/kg of the TPO mimetic peptide compound or placebo. In Cycle 3 to 6, the dose of the TPO mimetic peptide compound and placebo (i.e., dosing solution volume) will be adjusted based on the subject's Day 15 platelet count from the previous chemotherapy cycle as outlined in Table 11 to maximize safety, particularly with respect to elevated platelets. In all Cycles, the TPO mimetic peptide compound will not be administered if the platelet counts on the day of dosing exceed 700,000μ/L. Following chemotherapy administration, platelet counts are expected to progressively decline over approximately 2 weeks before starting to recover. Therefore, administering the TPO mimetic peptide compound on Day 1 of each chemotherapy cycle if a subject's platelet count is < 700,000 /μL is not an anticipated to be unsafe in the presence of chemotherapy agents known to result in thrombocytopenia. Subject safety will be further ensured by discontinuing a subject from the study if platelet count continues to be > 700,000/μL on Day 1 of two consecutive cycles.

An increase in hemoglobin with the use of ESAs has been associated with increased thrombovascular events. In order to further maximize subject safety, hemoglobin values for each subject will also be evaluated on Day 1 of each chemotherapy cycle to determine if the dose of the TPO mimetic peptide compound or placebo should be held. Specifically, if a subject has a hemoglobin value >15 g/dL or has an increase from baseline of ≥ 2 g/dL on Day 1 of any cycle, the subject will not be dosed with the TPO mimetic peptide compound for that given cycle. Subject safety will be further ensured by discontinuing a subject from the study if hemoglobin continues to be above >15 g/dL or there is ≥ 2 g/dL increase on Day 1 of two consecutive cycles. A hemoglobin value of 15 g/dL was chosen because it is approximately

the upper normal limit in healthy population. A ≥ 2 g/dL increase in hemoglobin above the baseline (but <15 g/dL) was chosen as it is considered a clinically significant event. The dose of the TPO mimetic peptide compound or placebo will not be modified based on hemoglobin values.

If a subject is not eligible to be dosed with the TPO mimetic peptide compound in any 2 consecutive chemotherapy cycles, he will not be considered evaluable and will be discontinued form the study. Frequent monitoring of coagulation parameters (e.g., PT, aPTT) will further assess subject safety and if necessary, subjects may be given prophylactic treatment with low dose aspirin.

Physical exams, vital signs, and ECGs will be assessed as a part of the safety evaluations. In addition, antibodies to the TPO mimetic peptide compound and huTPO will be evaluated over a one-year period. AEs, SAEs, and concomitant medications will be collected for up to 30 days following the last dose of the TPO mimetic peptide compound or placebo. SAEs and AEs beyond the 30 days post last dose of the TPO mimetic peptide compound or placebo will not be followed up as the disease progression in this subject population will make it difficult to assess the relationship of AEs to the TPO mimetic peptide compound beyond 30 days after the last dose of the TPO mimetic peptide compound.

Tumor assessments will be performed every two chemotherapy cycles (i.e., 6 weeks), or more frequently according to standard clinical practice for up to 6 months. This period is considered adequate to identify a potential trend for a deleterious effect of the TPO mimetic peptide compound on tumor growth in this subject population, as the median survival for this subject population is only 9 months. Progression-free survival will be evaluated at 6 months, and overall survival over a 2-year period, both will be considered exploratory safety evaluations.

Several specialized PD parameters will be evaluated. The measurement of coagulation parameters (Platelet Factor 4, prothrombin fragments 1+2, fibrin split product [D-dimer]; fibrinogen, and P-selectin) will assess the effect of the TPO mimetic peptide compound on functional coagulation. A reduction in fibrinogen levels may suggest prevention of development of microangiopathies that may also contribute to the prevention of chemotherapy-induced thrombocytopenia and anemia. These coagulation markers will enable assessment of any potential for increased thrombovascular events with increased platelets due to the TPO mimetic peptide compound treatment. Measurement of platelet derived growth factor-AA (PDGF-AA) and transforming growth factor- beta-1 ($TGF_{\beta 1}$) will be utilized to assess bone remodeling. Measurement of hematopoietic and thrombopoietic growth factors (serum huTPO and huEPO) will determine whether the TPO mimetic peptide compound has any affect on these growth factors, which are associated with maintenance and control of the level of the hemoglobin and platelet count endpoints.

PK samples will be collected to determine plasma concentrations of the TPO mimetic peptide compound and potentially determine a PK/PD relationship.

Subject Reported Outcomes assessments will be performed to explore the effects of the TPO mimetic peptide compound on a subject's daily function, fatigue, and other related measures. The Functional Assessment of Cancer Therapy-Anemia (FACT-An), Brief Fatigue Inventory (BFI), and Global Impression of Change (GIC) will be utilized for assessing subject reported outcomes.

A starting dose of 2.5 μg/kg in cycle 1 was chosen based on the results from an ongoing study in subjects with cancer, which investigated 1.5, 2.25 and 3 μg/kg doses.

Dose related platelet elevations are an expected pharmacological effect of the TPO mimetic peptide compound. As a result, at higher doses it is possible that excessive platelet elevations (beyond normal range) are observed. In the ongoing clinical study, at 1.5 and 2.25 μg/kg doses, none of the subjects had a ≥ 3-fold increase from baseline in platelet count. In contrast, at 3.0 μg/kg dose, two subjects had a ≥ 3-fold increase from baseline in platelet count around Day 15; the increase was transient and they were not considered adverse events. In the second cycle, after administration of the 3.0 μg/kg dose, the increase in platelet count observed was approximately 2-fold higher than baseline. This reduced elevation in platelet count relative to the increase in the first cycle was most likely due to cumulative myelosuppression as a result of continued chemotherapy. Elevated platelet counts of ≥ 3-fold increase from baseline have been observed in healthy subjects also at the 3.0 μg/kg dose. Therefore, it is planned to modify the doses of the TPO mimetic peptide compound for each subject in subsequent cycles (i.e., Cycle 2 to 6) based on the Day 15 platelet counts of the previous chemotherapy cycle, which is the anticipated to be the peak time for platelet count. This will allow optimal balancing of safe and efficacious doses. Therefore, a starting dose of 2.5 μg/kg body weight is considered appropriate. Based on the results from study, it is anticipated that due to cumulative myelosuppresion resulting from continued chemotherapy, reduced platelet elevation is expected to be observed in subsequent cycles with the same dose of the TPO mimetic peptide compound. Therefore, the dose in the second cycle is planned to be 3.0 μg/kg (unless the Day 15 platelet data from cycle 1 do not support an increase in the dose in the second cycle as explained earlier).

It is anticipated that due to cumulative bone marrow toxicity of chemotherapy, higher doses of the TPO mimetic peptide compound are likely to be needed to achieve the similar response in the subsequent cycles as the previous cycles. The dose titration range for Cycle 3 to 6 is 2.0 to 3.5 μg/kg. The highest dose of 3.5 μg/kg has been proposed in the event very low platelet counts are observed on Day 15 (i.e. < 50,000/μL).

### Individual Subject Stopping Criteria

An individual subject will be discontinued from the study if the following occurs at anytime during study participation:

- If platelet count continues to be > 700,000/μL on Day 1 of two consecutive cycles
- If hemoglobin continues to be above >15 g/dL or there is ≥ 2 g/dL increase on Day 1 of two consecutive cycles
- Subject is withdrawn for reasons such as below.

### Withdrawal From the Study

A subject may be withdrawn from the study for any of the following reasons:

- Tumor progression resulting in discontinuation of chemotherapy
- Discontinuation of study treatment. If a subject discontinues treatment before the end of the treatment phase, the first follow up visit should be performed.
- A subject may be discontinued from study treatment if:
    - The investigator believes that for safety reasons (e.g., adverse event) it is in the best interest of the subject to stop treatment
- Individual Stopping Criteria:
- If platelet count continues to be > 700,000/μL on Day 1 of two consecutive cycles
- Subject will be discontinued from the study if hemoglobin continues to be above >15 g/dL or there is ≥ 2 g/dL increase on Day 1 of two consecutive cycles
- The subject becomes pregnant
- If a subject is not dosed with the TPO mimetic peptide compound in any 2 consecutive chemotherapy cycles, the subject will be withdrawn from the study.
- Death

## DOSAGE AND ADMINISTRATION

On Day 1 of each chemotherapy cycle, subjects will receive an IV bolus dose of the TPO mimetic peptide compound or matching placebo (0.9% sodium chloride for injection) within 2 hours prior to receiving chemotherapy.

On Day 1 in Cycle 1, each subject will receive 2.5 μg/kg of the TPO mimetic peptide compound or placebo.

On Day 1 in Cycle 2, each subject will receive 3.0 μg/kg of the TPO mimetic peptide compound or placebo. In the event a subject's Cycle 1 Day 15 platelet count is >700,000/μL, and the platelet count remains >500,000/μL but is < 700,000/μL on Day 1 of Cycle 2, the subject will receive 2.5 μg/kg of the TPO mimetic peptide compound or placebo.

On Day 1 of Cycle 3 to 6, the dose of the TPO mimetic peptide compound or placebo (i.e., dosing solution volume) will be based on the subject's Day 15 platelet count in the previous cycle. The dose titration scheme for Cycle 3 to 6 is provided in Table 11.

### Gemcitabine, Carboplatin, and Cisplatin

The following chemotherapy regimens are required for eligibility:

- Gemcitabine: Dose = 1000 to 1250 mg/m$^2$; administered as a 30 minute infusion on Day 1 and Day 8 of each 21-day chemotherapy cycle
  *And, either*

- Carboplatin: Dose = 5 to 6 (target AUC of Carboplatin) x (GFR + 25); administered on Day 1 of each 21- day chemotherapy cycle
  *Or*
- Cisplatin: Dose = 75 to 80 mg/$m^2$; administered on Day 1 of each 21-day chemotherapy cycle

After completion of Cycle 1 (Visit 2), if necessary per clinical judgment, the chemotherapy doses may be modified according to the approved product label in the respective country of the investigative site.

## STUDY EVALUATIONS

### Overview

The approximate blood volume that will be collected for each subject is summarized in Table 13.
Blood samples will be collected from an intravenous cannula or by direct venipuncture. If an indwelling cannula is used for blood sample collection, a small amount of blood (i.e. no more than 1 mL) will be discarded each time a sample is taken via the cannula.

**Table 13.** Approximate Blood Volume Collected Per Subject

| Procedure | Blood volume per sample (mL) | Total number of samples | Total blood volume (mL) |
|---|---|---|---|
| Serology | 5 | 1 | 5 |
| Chemistry | 5 | 8 | 40 |
| Hematology | 4 | 21 | 84 |
| Coagulation | 2 | 21 | 42 |
| Serum iron, $B_{12}$, TIBC, ferritin | 7 | 1 | 7 |
| Antibody formation to the TPO mimetic peptide compound | 3 | 2 | 6 |
| Antibody formation to huTPO | 3 | 2 | 6 |
| PK | 1 | 8 | 8 |
| Fibrinogen, F1+2, D-dimer | 4.5 | 3 | 13.5 |
| PF4 | 2.7 | 3 | 8.1 |
| Soluble P-selectin, $TGF_\beta$, PDGF | 4 | 3 | 12 |
| Serum huTPO | 1.5 | 3 | 4.5 |
| Serum huEPO | 1.5 | 3 | 4.5 |
| ***Approximate* Subtotal** | | | **~ 241 mL** |
| Serum pregnancy test (women of childbearing potential) | 5 | 2 | 10 |
| ***Approximate* Total** | | | **~ 263 mL** |

### Visit 2 to 19: Double-Blind Treatment Phase

Subjects will arrive at the investigative center on the morning of Day 1, 8, and 15 of each 21-day chemotherapy cycle for up to 6 cycles (Cycle 1 to Cycle 6).
Visit 2, 5, 8, 11, 14, and 17 correspond to Day 1 of each chemotherapy cycle (Cycle 1 to 6).
Visit 3, 6, 9, 12, 15, and 18 correspond to Day 8 of each chemotherapy cycle (Cycle 1 to 6). Each of these visits may be conducted +/- 2 days.
Visit 4, 7, 10, 13, 16, and 19 correspond to Day 15 of each chemotherapy cycle (Cycle 1 to 6). Each of these visits may be conducted +/- 2 days.
Predose is defined as within 2 hours of study medication administration. The pharmacokinetic sample scheduled for predose should be taken as close to the dosing time as possible. The Visit 2 predose results will be considered the subject's baseline values for statistical analyses.

**Efficacy**

**Evaluations and Criteria**

***Primary***

The primary efficacy evaluation will be the difference in incidence rates between the TPO mimetic peptide compound and placebo on the composite endpoint of Grade 2 or higher anemia, or a ≥ 2 g/dL drop in hemoglobin on the first day of any chemotherapy cycle (Cycle 2 to 6) relative to baseline (Cycle 1, Day 1), or the use of rescue intervention for anemia.

Hemoglobin and the use of ESAs and RBC transfusions will be the criteria used to evaluate the primary efficacy endpoint. These parameters will also be part of the safety evaluation.

Anemia will be graded based on the *Common Terminology Criteria for Adverse Events (CTCAE): Version 3.0.*[55] CTCAE Version 3.0 criteria for hemoglobin can be found in Attachment 1.

***Secondary***

The secondary efficacy endpoints are:

- The difference between the TPO mimetic peptide compound OUND and placebo on the incidence rates on the composite endpoint of Grade 2 or higher thrombocytopenia or the use of platelet transfusion.
- The difference between the TPO mimetic peptide compound and placebo on the incidence rates of each individual component of the composite endpoint for anemia and thrombocytopenia.

Hemoglobin, platelet count, use of ESAs, and the use of RBC and platelet transfusions will be the criteria used to evaluate the secondary efficacy endpoints. These parameters will also be part of the safety evaluation.

Thrombocytopenia and anemia will be graded based on the *Common Terminology Criteria for Adverse Events (CTCAE): Version 3.0.*[55]

**Pharmacokinetic Evaluations**

Venous blood samples of 1 mL will be collected for determination of the TPO mimetic peptide compound plasma concentrations.

**Pharmacokinetic Parameters**

Individual plasma concentration data from each sample time point will be determined.

**Pharmacodynamic Evaluations**

The following PD evaluations will be performed:

- ***Coagulation parameters:*** Fibrinogen, platelet factor 4 (PF4), prothrombin fragments 1 + 2 (PF1+2), fibrinogen degradation product (D-dimer), and P-selectin. These parameters will also be a safety evaluation.
- ***Marker for bone remodeling:*** Platelet derived growth factor-AA (PDGF-AA) and transforming growth factor- beta-1 ($TGF_{\beta 1}$). These parameters will also be a safety evaluation.
- ***Human growth factors:*** Serum native human thrombopoietin (huTPO) and erythropoietin (huEPO) concentrations.

**Laboratory Tests**

Venous blood samples for serum chemistry, hematology, coagulation, and a random urine sample for urinalysis will be collected. Several laboratory tests (e.g., hemoglobin, platelet count) will also be efficacy evaluations.

**Hematology**

Hemoglobin
Hematocrit
Platelet count
Red blood cell count
Percent reticulocytes
White blood cell count with differential
Red blood cell indices (MCHC, MCV, MCH, RDW)

**Urinalysis - Sediment (Performed only if dipstick is abnormal)**

*If dipstick result is abnormal, flow cytometry will be used to measure sediment. In case of discordance between the dipstick results and the flow cytometric results, the sediment will be examined microscopically.*

| | |
|---|---|
| Red blood cells | Crystals |
| White blood cells | Casts |
| Epithelial cells | Bacteria |

**Other**

| | |
|---|---|
| Iron | Folate |
| $B_{12}$ | TIBC |

Ferritin

**Antibody Formation**

Venous blood samples will be obtained to determine the antibody titers against huTPO and the TPO mimetic peptide compound. Serum will be analyzed.

**Tumor Response Assessment**

Tumor assessment will be performed and evaluated per RECIST criteria. [43,44]
The same procedure (e.g., CT, MRI) used for tumor assessment at Screening must be used throughout the entire study.

**Progression-Free Survival**

Tumor response assessment will allow an evaluation of progression free survival at 6 months.
Progression free survival (PFS), computed for all randomized subjects, is defined as the time from randomization until the time of disease progression is first documented. Subjects who die without a reported prior progression will be considered to have progressed on the date of their death. Subjects who have not progressed or died will be censored on the date of their last tumor assessment (i.e., 6 months from first dose of study medication).

**Overall Survival**

Overall survival, computed for all randomized subjects, is defined as the date of first dosing until the time death is documented. Overall survival will be assessed over a period of 24 months from first dose of study medication. Subjects who have not died will be censored on the date of the last follow up visit.

**Other Safety Evaluations**

The following will be evaluated throughout the study:

- Incidence of the use of rescue intervention for anemia or thrombocytopenia
- Incidence of a delay in administration or a dose reduction in chemotherapy
- Incidence of a dose reduction in study medication dose
- Incidence of thrombovascular events (TVE)

**Functional Assessment of Cancer Therapy - Anemia (FACT-An)**

The Functional Assessment of Cancer Therapy - Anemia (FACT-An) is a 47-item subject reported outcome measure that was developed to assess fatigue and anemia related concerns in people with cancer. It includes the FACT-General (FACT-G) consisting of physical, functional, emotional, and social/family well-being subscales (27 items), and the anemia scale consisting of fatigue (13 items) and non fatigue (7 items) subscales.[49, 50] The FACT items are rated on a 5-point Likert-type scale ranging from 0 "not at all" to 4 = "very much". Higher scores represent better health status or less severe symptoms. FACT-An scores have been shown to be associated with hemoglobin levels in subjects undergoing chemotherapy.[48]

**Brief Fatigue Inventory (BFI)**

The BFI was developed and validated with subjects with cancer experiencing treatment or disease-related anemia.[51]

The BFI includes 3 items that address fatigue severity (weariness, tiredness) "now", "usual level of fatigue over last 24 hours" and "worst level of fatigue over the last 24 hours." An additional 6 items assess the extent to which fatigue interferes with general activity, mood, walking ability, normal work, relations with other people, and enjoyment of life. Each item includes an 11-point numeric rating scale ranging from 0 (no fatigue or interference) to 10 (as bad as you can imagine or completely interferes).

**Global Impression of Change (GIC)**

For purposes of further validation of the BFI, subjects will be asked provide an assessment of their global impression of change in fatigue using a single item measure. This measure is included in order to associate observed changes in the BFI to subject perceptions of change in fatigue. Specifically this will assist in evaluating the responsiveness and clinically meaningfulness of observed change in the BFI.

**Data Analysis**

**Efficacy Analyses**

Efficacy analyses will be performed on all subjects receiving at least one dose of the TPO mimetic peptide compound or placebo and with at least one efficacy assessment.

**Primary**

The primary efficacy evaluation will be the difference in incidence rates between the TPO mimetic peptide compound and placebo on the composite endpoint of Grade 2 or higher anemia, or a $\geq$ 2 g/dL drop in hemoglobin on the first day of any chemotherapy cycle (Cycle 2 to 6) relative to baseline (Cycle 1, Day 1), or the use of rescue intervention for anemia.

In order to account for potential differences in follow up, the composite endpoint incidence rates for the active and placebo groups will be estimated using the Kaplan-Meier approach, with Greenwood formula estimates of the standard deviations. A 90% (two-sided) confidence interval on the difference in incidence rates will be provided. As an additional sensitivity analysis, the time to reach the composite endpoint will also be evaluated using the relative risk estimate from the Cox regression model with baseline hemoglobin level (predose at Visit 2) as a covariate and disease stage and platinum chemotherapy regimen as factors in the model.

**Secondary**

The secondary efficacy evaluations are:

- The difference in incidence rates between the TPO mimetic peptide compound and placebo on the composite endpoint of Grade 2 or higher thrombocytopenia or the use of platelet transfusion.

- The difference between the TPO mimetic peptide compound and placebo on the incidence rates of each individual component of the composite endpoint for anemia and thrombocytopenia.

The secondary efficacy evaluation of the incidence rates of the composite endpoint for thrombocytopenia (Grade 2 or higher thrombocytopenia or the use of platelet transfusion) will be analyzed as described above for the primary efficacy evaluation.

Each component of the composite endpoints for anemia and thrombocytopenia will also be evaluated in a similar fashion.

**Pharmacokinetics**

Data will be listed for all subjects with available plasma concentrations per treatment. All concentrations below the limit of quantification (LOQ) or missing data will be labeled as such in the concentration data listings. Concentrations below the LOQ will be treated as zero in the summary statistics. All subjects and samples excluded from the analysis will be clearly documented in the study report.

Data for all subjects receiving at least one dose of active TPO mimetic peptide compound will be included in the pharmacokinetic analyses. Descriptive statistics (including means, median, standard deviations and coefficients of variation) of concentration data will be generated for each dose.

**Pharmacodynamic Analyses**

Pharmacodynamic analyses will be performed on all subjects receiving at least one dose of the TPO mimetic peptide compound or placebo and with at least one pharmacodynamic assessment. Summary statistics will be generated for all pharmacodynamic parameters.

**Pharmacokinetic/Pharmacodynamic Analyses**

Where appropriate, plasma and blood concentrations and corresponding pharmacodynamic measurements will be plotted to evaluate their relationship.

**TPO MIMETIC PEPTIDE COMPOUND INFORMATION**

**Physical Description of the TPO Mimetic Peptide Compound**

| | |
|---|---|
| *Strength:* | 2.0 mg/mL solution, after reconstitution |
| *Dosage form:* | Intravenous solution |
| *Placebo:* | Intravenous 0.9% saline for injection |

The TPO mimetic peptide compound will be provided as a lyophilized powder for reconstitution (2.0 mg/mL solution *after* reconstitution). The lyophilized powder (5 mg PEGylated peptide) is in a single-use 3- or 4 mL glass vial.

**REFERENCES**

1. Ludwig H, Van Belle S, Barrett-Lee P, Birgegard G, Bokemeyer C, Gascon P, Kosmidis P, Krzakowski M, Nortier J, Olmi P, Schneider M, Schrijvers D. The European cancer anaemia survey (ECAS): A large, multinational, prospective survey defining the prevalence, incidence, and treatment of anaemia in cancer subjects. European Journal of Cancer 2004; 40: 2293-2306.

2. Groopman JE, Itri LM. Chemotherapy-induced anemia in adults: incidence and treatment. J Natl Cancer Inst 1999; 91: 1616-1634.

3. Ohe Y, Ohashi Y, Kubota K, Tamura T, Nakagawa K, Negoro S, Nishiwaki Y, Saijo N, Ariyoshi Y, Fukuoka M. Randomized phase III study of cisplatin plus irinotecan versus carboplatin plus paclitaxel, cisplatin plus gemcitabine, and cisplatin plus vinorelbine for advanced non-small-cell lung cancer; Four-Arm Cooperative study in Japan. Annals of Oncology 2007; 18: 317-323.

4. Schiller JH, Harrington D, Belani CP, Langer C, Sandler A, Krook J, Zhu J, Johnson DH. Comparison of Four Chemotherapy Regimens for Advanced Non-Small Cell Lung Cancer; N Engl J Med, 2002; 346(2): 92-98.

5. Gemzar (Gemcitabine HCL) for Injection Label; www.FDA.gov

6. Wilson J, Yao GL, Raftery J, et al. A systematic review and economic evaluation of epoetin alfa, epoetin beta, and darbepoetin alfa in anemia associated with cancer, especially that attributable to cancer treatment. Health Technol Assess, 2007; 11(13): 1-220

7. Rizzo JD, Lichten AE, Woolf SH, et al. Use of epoetin in subjects with cancer: evidence-based clinical practice guidelines of the American Society of Clinical Oncology and the American Society of Hematology. J Clin Oncol 2002; 20: 4083-4107.

8. National Comprehensive Cancer Network. Cancer and treatment-related anemia. Available at: http://www.nccn.org/professionals/physician_gls/PDF/anemia.pdf. Accessed 08 August 2005.

9. Bokemeyer C, Aapro MS, Courdi A, et al. EORTC guidelines for the use of erythropoietic proteins in anaemic subjects with cancer. Eur J Cancer 2004; 40: 2201-2216.

10. Rizzo JD, Somerfield MR, Hagerty KL, et al. American Society of Clinical Oncology/American Society of Hematology 2007 Clinical Practice Guideline Update on the Use of Epoetin and Darbepoetin. J Clin Oncol 2007; 1-17 (Epub Ahead of Print).

11. Available at: http://www.fda.gov/cder/approval/index.htm. Accessed 08 August 2005.

12. Murone M., Carpenter DA., de Sauvage FJ. Hematopoietic deficiencies in c-mpl and TPO knockout mice. Stem Cells 1998; 16: 1-6.

13. Ballmaier M., Germeshausen M, Schulze H, et al. c-mpl mutations are the cause of congenital amegakaryocytic thrombocytopenia. Blood 2001; 97: 139-146.

14. Borge OJ, Ramsfjell V, Cui L, Jacobsen SEW. Ability of early acting cytokines to directly promote survival and suppress apoptosis of human primitive CD34+CD38-bone marrow cells with multilineage potential at the single-cell level: key role of thrombopoietin. Blood 1997; 90: 2282-2292.

15. Mouthon MA, Van der Meeren A, Gauler MH, Visser TP, Squiban C, Gourmelon P, Wagemaker G. Thrombopoietin promotes hematopoietic recovery and survival after high-dose whole body irradiation. Int J Radiat Oncol Biol Phys 1999; 43: 867-875.

16. Drouet M, Mathieu J, Grenier N, Multon E, Sotto JJ, Herodin F. The reduction of in vitro radiation-induced Fas-related apoptosis in CD34+ progenitor cells by SCF, FLT-3 ligand, TPO, and IL-3 in combination resulted in CD34+ cell proliferation and differentiation. Stem Cells 1999; 17: 273-285.

17. Ramsfjell V, Borge OJ, Cui L, Jacobsen SE. Thrombopoietin directly and potently stimulates multilineage growth and progenitor cell expansion from primitive (CD34+ CD38-) human bone marrow progenitor cells: distinct and key interactions with the ligands for c-kit and flt3, and inhibitory effects of TGF-beta and TNF-alpha. J Immunol 1997; 158: 5169-5177.

18. Neelis KJ, Visser TP, Dimjati W, et al. A single dose of thrombopoietin shortly after myelosuppressive total body irradiation prevents pancytopenia in mice by promoting short-term multilineage spleen-repopulating cells at the transient expense of bone marrow-repopulating cells. Blood 1998; 92: 1586-1597.

19. Torii Y, Nitta Y, Akahori H, et al. Mobilization of primitive haemopoietic progenitor cells and stem cells with long-term repopulating ability into peripheral blood in mice by pegylated recombinant human megakaryocyte growth and development factor. Br J Haematol 1998; 103: 1172-1180.

20. Elting LS, Rubenstein EB, Martin CG, et al. Incidence, cost, and outcomes of bleeding and chemotherapy dose modification among solid tumor subjects with chemotherapy-induced thrombocytopenia. J Clin Oncol 2001; 19: 1137-1146.

21. Li J, Yang C, Xia Y, et al. Thrombocytopenia caused by the development of antibodies to thrombopoietin. Blood 2001; 98: 3241-3248.

22. Columbyova L, Massima L, Scadden D.T. Thrombopoietin receptor expression in human cancer cell lines and primary tissues: Cancer Res. 1995; 55: 3509-3512.

23. Investigator's Brochure - The TPO mimetic peptide compound; November 2007.

24. Jemal A, Siegel R, Ward E, Murray T, Xu J, Thun MJ: Cancer statistics, 2007. CA Cancer J Clin 57:43-66, 2007.

25. AJCC Cancer Staging Manual, 6 edition. New York, Springer-Verlag, 2002

26. Belani CP, Larocca RV, Rinaldi WJ: A multicenter, phase III randomized trial for stage IIIB/IV NSCLC of weekly paclitaxel and carboplatin vs. standard paclitaxel and carboplatin given every three weeks, followed by weekly paclitaxel. Proc Am Soc Clin Oncol 23:619, 2004

27. Belani CP, Lee JS, Socinski MA, Robert F, Waterhouse D, Rowland K, Ansari R, Lilenbaum R, Natale RB: Randomized phase III trial comparing cisplatin-etoposide to carboplatin-paclitaxel in advanced or metastatic non-small cell lung cancer. Ann Oncol 16:1069-75, 2005

28. Cardenal F, Lopez-Cabrerizo MP, Anton A, Alberola V, Massuti B, Carrato A, Barneto I, Lomas M, Garcia M, Lianes P, Montalar J, Vadell C, Gonzalez-Larriba JL, Nguyen B, Artal A, Rosell R: Randomized phase III study of gemcitabine-cisplatin versus etoposide-cisplatin in the treatment of locally advanced or metastatic non-small-cell lung cancer. J Clin Oncol 17:12-8, 1999

29. Fossella F, Pereira JR, von Pawel J, Pluzanska A, Gorbounova V, Kaukel E, Mattson KV, Ramlau R, Szczesna A, Fidias P, Millward M, Belani CP: Randomized, multinational, phase III study of docetaxel plus platinum combinations versus vinorelbine plus cisplatin for advanced non-small-cell lung cancer: the TAX 326 study group. J Clin Oncol 21:3016-24, 2003

30. Kelly K, Crowley J, Bunn PA, Jr., Presant CA, Grevstad PK, Moinpour CM, Ramsey SD, Wozniak AJ, Weiss GR, Moore DF, Israel VK, Livingston RB, Gandara DR: Randomized phase III trial of paclitaxel plus carboplatin versus vinorelbine plus cisplatin in the treatment of subjects with advanced non--small-cell lung cancer: a Southwest Oncology Group trial. J Clin Oncol 19:3210-8, 2001

31. Ohe Y, Ohashi Y, Kubota K, Tamura T, Nakagawa K, Negoro S, Nishiwaki Y, Saijo N, Ariyoshi Y, Fukuoka M: Randomized phase III study of cisplatin plus irinotecan versus carboplatin plus paclitaxel, cisplatin plus gemcitabine, and cisplatin plus vinorelbine for advanced non-small-cell lung cancer: Four-Arm Cooperative Study in Japan. Ann Oncol 18:317-23, 2007

32. Rudd R, Gower N, James L: Phase III randomised comparison of gemcitabine and carboplatin with mitomycin, ifosfamide and cisplatin in advanced non-small cell lung cancer. Proc Am Soc Clin Oncol 21:(Abstract 1164), 2002

33. Sandler AB, Nemunaitis J, Denham C, von Pawel J, Cormier Y, Gatzemeier U, Mattson K, Manegold C, Palmer MC, Gregor A, Nguyen B, Niyikiza C, Einhorn LH: Phase III trial of gemcitabine plus cisplatin versus cisplatin alone in subjects with locally advanced or metastatic non-small-cell lung cancer. J Clin Oncol 18:122-30, 2000

34. Sederholm C, Hillerdal G, Lamberg K, Kolbeck K, Dufmats M, Westberg R, Gawande SR: Phase III trial of gemcitabine plus carboplatin versus single-agent gemcitabine in the treatment of locally advanced or metastatic non-small-cell lung cancer: the Swedish Lung Cancer Study Group. J Clin Oncol 23:8380-8, 2005

35. Smit EF, van Meerbeeck JP, Lianes P, Debruyne C, Legrand C, Schramel F, Smit H, Gaafar R, Biesma B, Manegold C, Neymark N, Giaccone G: Three-arm randomized study of two cisplatin-based regimens and paclitaxel plus gemcitabine in advanced non-small-cell lung cancer: a phase III trial of the European Organization for Research and Treatment of Cancer Lung Cancer Group--EORTC 08975. J Clin Oncol 21:3909-17, 2003

36. Wozniak AJ, Crowley JJ, Balcerzak SP, Weiss GR, Spiridonidis CH, Baker LH, Albain KS, Kelly K, Taylor SA, Gandara DR, Livingston RB: Randomized trial comparing cisplatin with cisplatin plus vinorelbine in the treatment of advanced non-small-cell lung cancer: a Southwest Oncology Group study. J Clin Oncol 16:2459-65, 1998

37. Abratt RP, Hart GJ: 10-year update on chemotherapy for non-small cell lung cancer. Ann Oncol 17 Suppl 5:v33-v36, 2006.

38. Sandler A, Gray R, Perry MC, Brahmer J, Schiller JH, Dowlati A, Lilenbaum R, Johnson DH: Paclitaxel-carboplatin alone or with bevacizumab for non-small-cell lung cancer. N Engl J Med 355:2542-50, 2006.

39. Manegold C, von Pawel J, Zatloukal P: Randomised, double-blind multicentre phase III study of bevacizumab in combination with cisplatin and gemcitabine in chemotherapy-naive subjects with advanced or recurrent non-squamous non-small cell lung cancer (NSCLC): B017704. Proc Am Soc Clin Oncol 25:(Abstract 7514), 2007

40. Sanborn RE, Sandler AB: The safety of bevacizumab. Expert Opin Drug Saf 5:289-301,2006.

41. Langer C, Li S, Schiller J, Tester W, Rapoport BL, Johnson DH: Randomized phase II trial of paclitaxel plus carboplatin or gemcitabine plus cisplatin in Eastern Cooperative Oncology Group performance status 2 non-small-cell lung cancer subjects: ECOG 1599. J Clin Oncol 25:418-23, 2007

42. Wang LR, Huang MZ, Zhang GB, Xu N, Wu XH: Phase II study of gemcitabine and carboplatin in subjects with advanced non-small-cell lung cancer. Cancer Chemother Pharmacol 60:601-7, 2007

43. Therasse P, Arbuck S, Eisenhauer EA, et al. New guidelines to evaluate the response to treatment in solid tumors. J Natl Cancer Inst 92 (3): 205-16, 2000.

44. RECIST guidelines available at http://ctep.cancer.gov/guidelines/recist.html Accessed 05-November-2007.

45. Bokemeyer C, Aapro MS, et al. EORTC guidelines for the use of erythropoietic proteins in anaemic subjects with cancer: 2006 update. Eur J Cancer 2007;43(2): 258-70.

46. National Comprehensive Cancer Network® Clinical Practice Guidelines in Oncology™ Cancer and Treatment Related Anemia V.3.2007 available at www.nccn.org

47. Schiffer CA, Anderson KC, Bennett CL, et al. Platelet transfusion for subjects with cancer: clinical practice guidelines of the American society of clinical oncology. J Clin Oncol 19 (5): 1519-38, 2001.

48. Oken, M.M., Creech, R.H., Tormey, D.C., Horton, J., Davis, T.E., McFadden, E.T., Carbone, P.P.: Toxicity And Response Criteria Of The Eastern Cooperative Oncology Group. Am J Clin Oncol 5:649-655,1982.

49. Yellen SB, Cella DF, Webster K, Blendowski C, Kaplan E. Measuring fatigue and other anemia-related symptoms with the Functional Assessment of Cancer Therapy (FACT) measurement system. J Pain Symptom Manage 1997 Feb; 13(2):63-74

50. Cella D, Webster K. Linking outcomes management to quality-of-life measurement. Oncology (Williston Park). 1997 Nov; 11(11A): 232-5.

51. Mendoza TR, Wang XS, Cleeland CS, Morrissey M, Johnson BA, Wendt JKI, Huber SL. The rapid assessment of fatigue severity in cancer subjects: use of the Brief Fatigue Inventory. Am Cancer Soc 1999;85: 1186-96

52. Crino L, Scagliotti GV, et al. Gemcitabine and cisplatin versus mitomycin, ifosfamide, and cisplatin in advanced non-small-cell lung cancer: a randomized phase III study of the Italian lung cancer project. J Clin Oncol 17(11), 1999; 3522-30.

53. Cella D, Eton DT, Lai Jin-Shei, Peterman AH, Merkel DE. Combining Anchor and distribution-based methods to derive minimal clinically important differences on the Functional Assessment of Cancer Therapy (FACT) Anemia and Fatigue Scales. J Pain and Symptom Management 2002; 24(6):547-561.

54. Lokich J, Anderson N. Carboplatin versus cisplatin in solid tumors: An analysis of the literature. Annals of Oncology 9: 1998, 13-21.

55. Cancer Therapy Evaluation Program. Common Terminology Criteria for Adverse Events Version 3.0, DCTD NCI NIH DHHS. 2006; 1-72. http://ctep. cancer. gov/forms/CTCAEv3 .pdf

**Attachment 1**

Assessment of Hemoglobin and Platelet Count Using the Common Terminology Criteria for Adverse Events (Version 3.0)

| **Common Terminology Criteria for Adverse Events (CTCAE): Version 3.0** | | | |
|---|---|---|---|
| **Grade** | **Definition** | **Hemoglobin** | **Platelets** |
| 0 | No Adverse Event or within normal limits | WNL | WNL |
| 1 | Mild Adverse Event | < LLN - 10.0 g/dL<br><LLN - 6.2 mmol/L<br><LLN - 100 g/L | <LLN - 75,000/$mm^3$<br><LLN - 75.0 x $10^9$/ L |
| 2 | Moderate Adverse Event | < 10.0 - 8.0 g/dL<br>< 6.2 - 4.9 mmol/L<br>< 100 - 80 g/L | < 75,000 - 50,000/$mm^3$<br>< 75.0 - 50.0 x $10^9$/ L |
| 3 | Severe and undesirable Adverse Event | < 8.0 - 6.5 g/dL<br>< 4.9 - 4.0 mmol/L<br>< 80 - 65 g/L | < 50,000 - 25,000/$mm^3$<br>< 50.0 - 25.0 x $10^9$/ L |
| 4 | Life-threatening or disabling adverse event | < 6.5 g/dL<br>< 4.0 mmol/L<br>< 65 g/L | < 25,000/$mm^3$<br>< 25.0 x $10^9$/ L |
| 5 | Death related to Adverse Event | Death | Death |

**Claims**

1. A TPO mimetic peptide compound for use in a method for treating and/or preventing chemotherapy induced anemia in a subject undergoing treatment for cancer, the method comprising:

administering the TPO mimetic peptide compound within a specified time frame surrounding administration of said cancer treatment,
wherein said TPO mimetic peptide compound has the following structure:

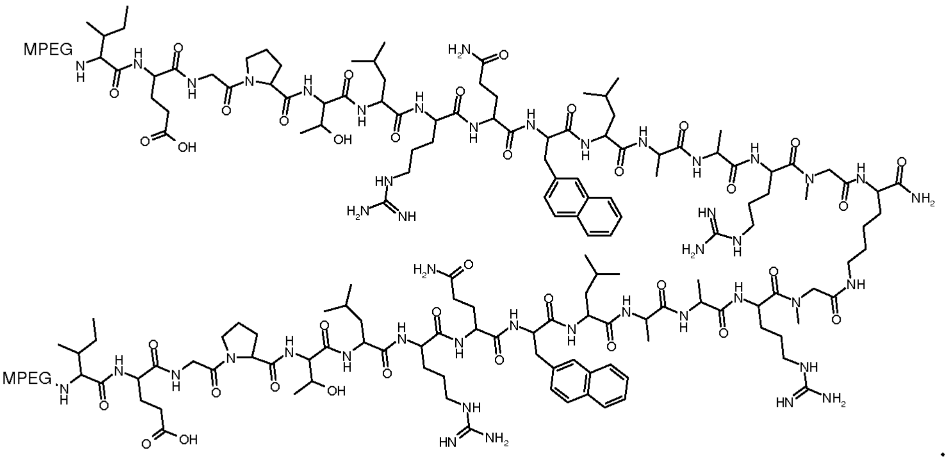

and wherein said specified time frame is within two hours prior to said administration of said cancer treatment.

2. The TPO mimetic peptide compound for use according to claim 1, wherein said treatment is administration of a chemotherapeutic agent.

3. The TPO mimetic peptide compound for use according to claim 2, wherein said chemotherapeutic agent is a platinum-based chemotherapeutic agent.

4. The TPO mimetic peptide compound for use according to claim 3, wherein said platinum-based chemotherapeutic agent is selected from the group consisting of carboplatin and cisplatin.

## Patentansprüche

1. TPO-Mimetikpeptidverbindung zur Verwendung in einem Verfahren zur Behandlung und/oder Prävention einer durch Chemotherapie induzierten Anämie bei einem Patienten, der sich einer Krebsbehandlung unterzieht, das Verfahren umfassend:

   Verabreichen der TPO-Mimetikpeptidverbindung innerhalb eines bestimmten Zeitrahmens um die Verabreichung der Krebsbehandlung,
   wobei die TPO-Mimetikpeptidverbindung die folgende Struktur hat:

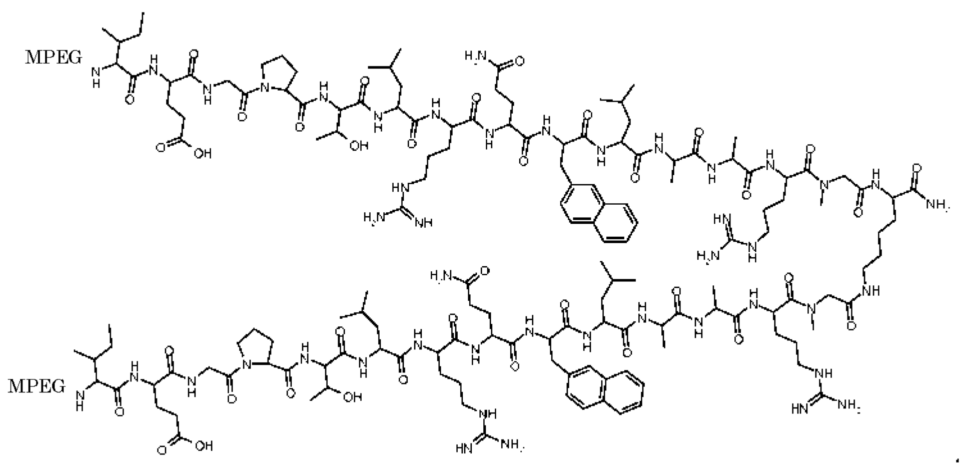

   und wobei der bestimmte Zeitrahmen innerhalb von zwei Stunden vor der Verabreichung der Krebsbehandlung liegt.

2. TPO-Mimetikpeptidverbindung zur Verwendung nach Anspruch 1, wobei die Behandlung die Verabreichung eines

Chemotherapeutikums ist.

3. TPO-Mimetikpeptidverbindung zur Verwendung nach Anspruch 2, wobei das Chemotherapeutikum ein Platin-basiertes Chemotherapeutikum ist.

4. TPO-Mimetikpeptidverbindung zur Verwendung nach Anspruch 3, wobei das Platin-basierte Chemotherapeutikum ausgewählt ist aus der Gruppe bestehend aus Carboplatin und Cisplatin.

## Revendications

1. Un composé consistant en un peptide mimétique de TPO pour être utilisé dans un procédé de traitement et/ou de prévention d'une anémie induite par chimiothérapie chez un sujet soumis à un traitement anticancéreux, comprenant :

   - l'administration d'un composé peptidique mimétique de TPO dans un laps de temps spécifié entourant l'administration dudit traitement anticancéreux,

   dans lequel ledit composé peptidique mimétique de TPO présente la structure suivante:

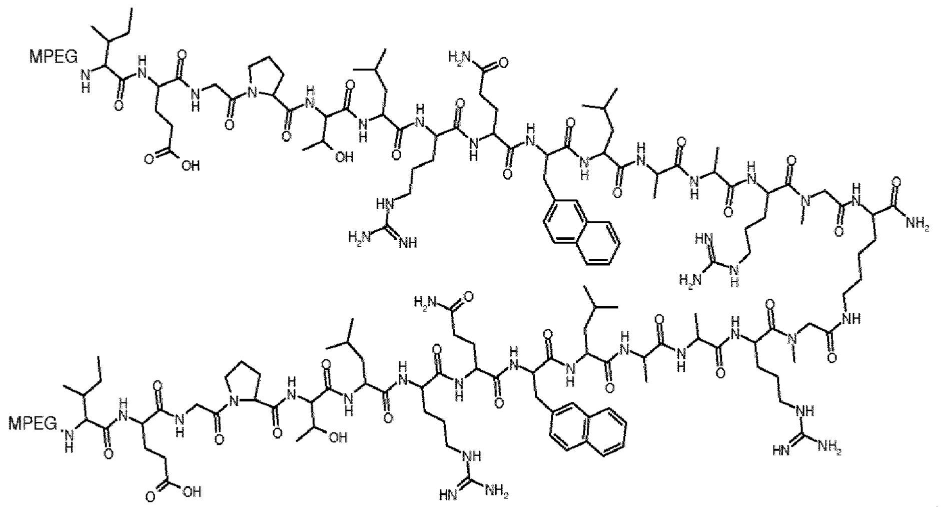

   et dans lequel ledit laps de temps spécifié est dans les deux heures précédent ladite administration du traitement anticancéreux.

2. Le composé consistant en un peptide mimétique de TPO pour être utilisé selon la revendication 1, dans lequel ledit traitement est l'administration d'un agent chimio-thérapeutique.

3. Le composé consistant en un peptide mimétique de TPO pour être utilisé selon la revendication 2, dans lequel ledit agent chimio-thérapeutique est un agent chimio-thérapeutique à base de platine.

4. Le composé consistant en un peptide mimétique de TPO pour être utilisé selon la revendication 3, dans lequel ledit agent chimio-thérapeutique à base de platine est choisi dans le groupe consistant en carboplatine et cisplatine.

Figure 1. Individual Times to Endpoint for Mice in the HT29-e141 Study

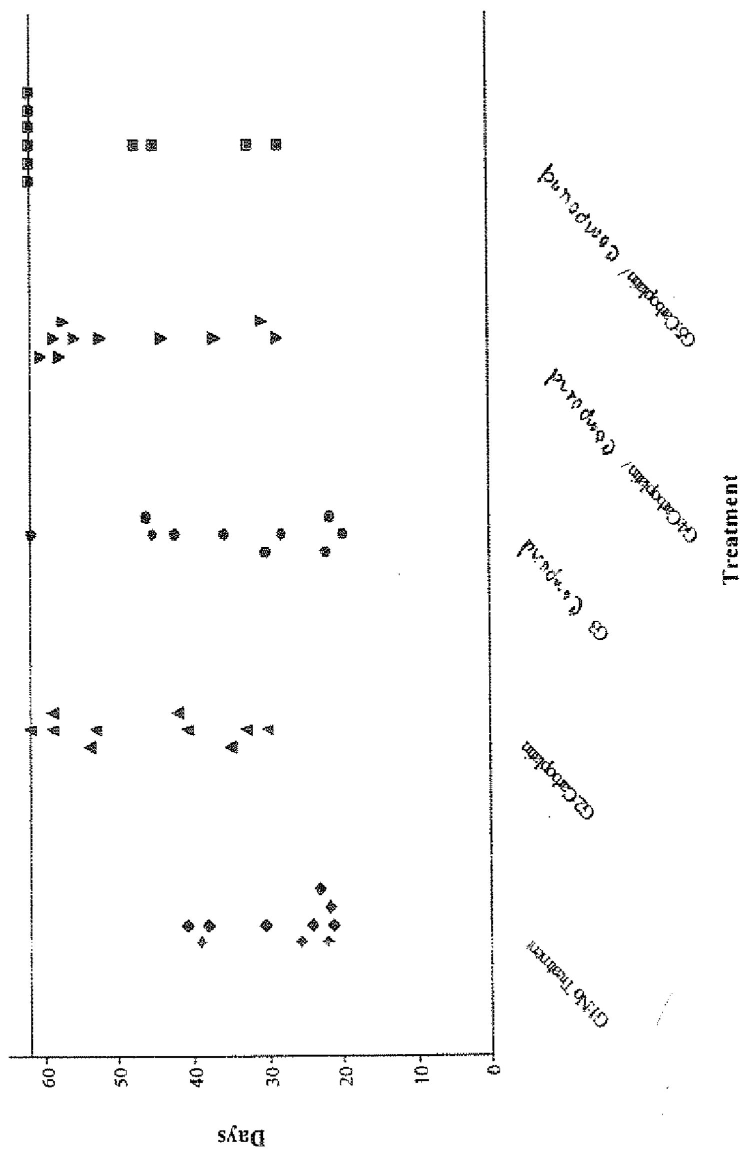

# Figure 2. Median Tumor Growth and Kaplan-Meier Plot for the HT29-e141 Study

Figure 2a

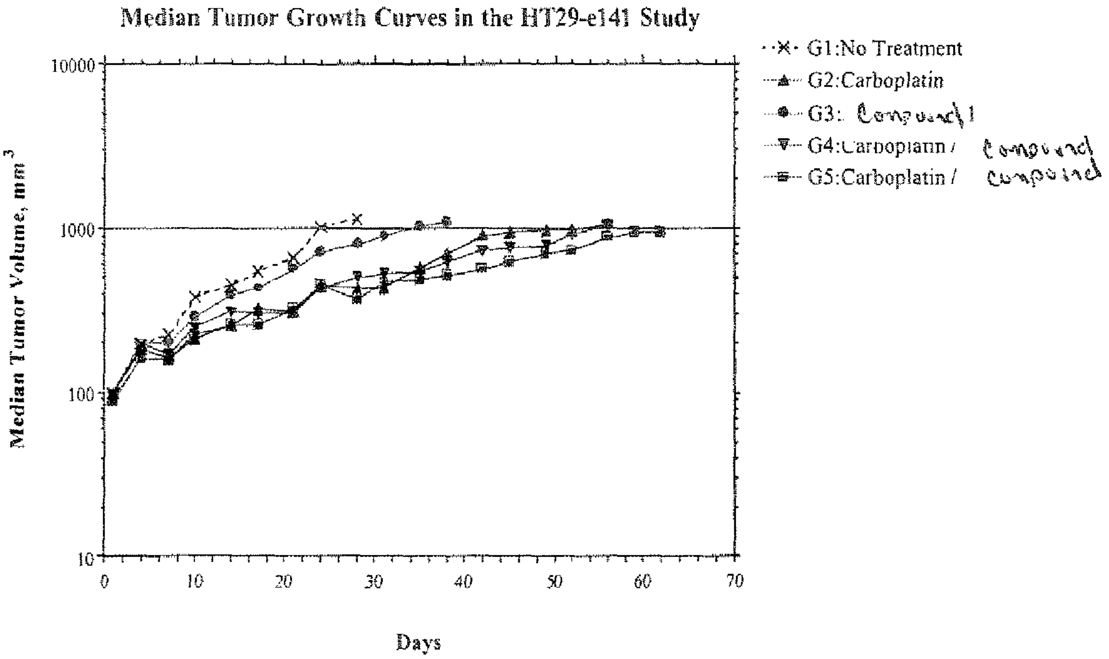


Figure 2b

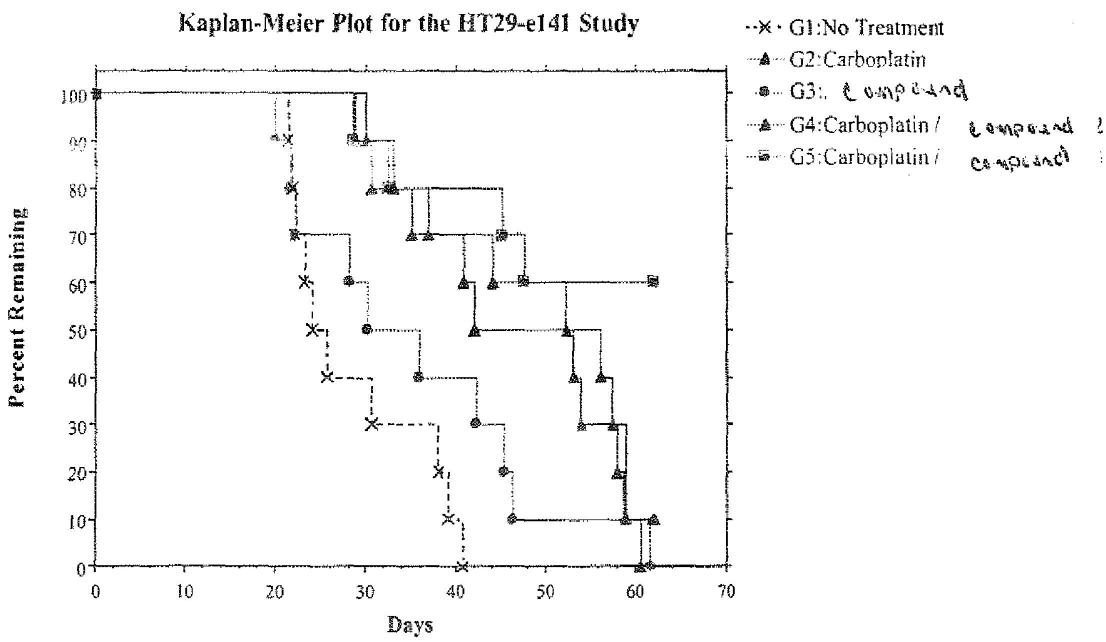

Figure 3a. Mean Platelet Counts in the HT29-e141 Study

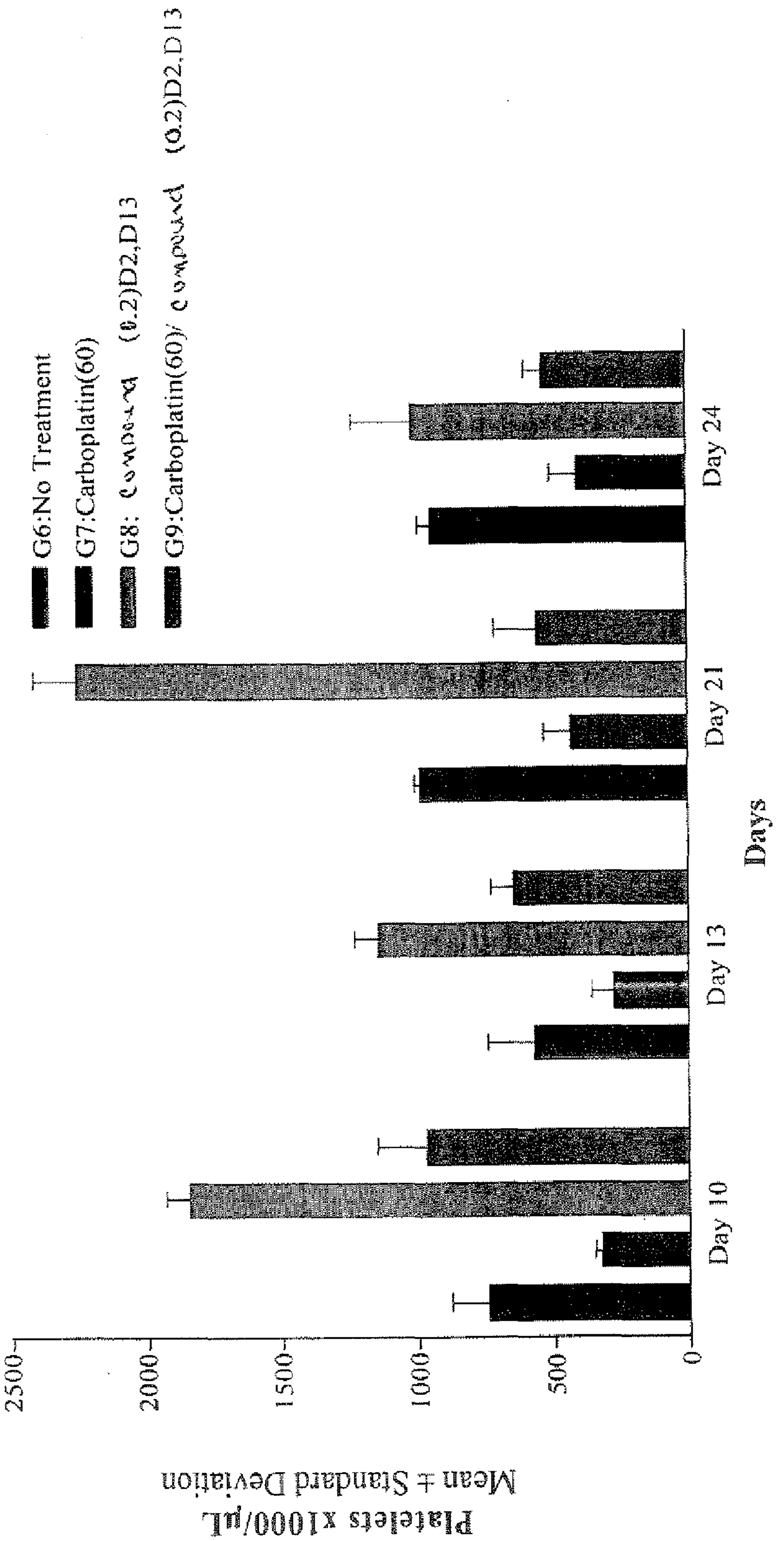

**Figure 3b. Mean Reticulocyte Values in the HT29-e141 Study**

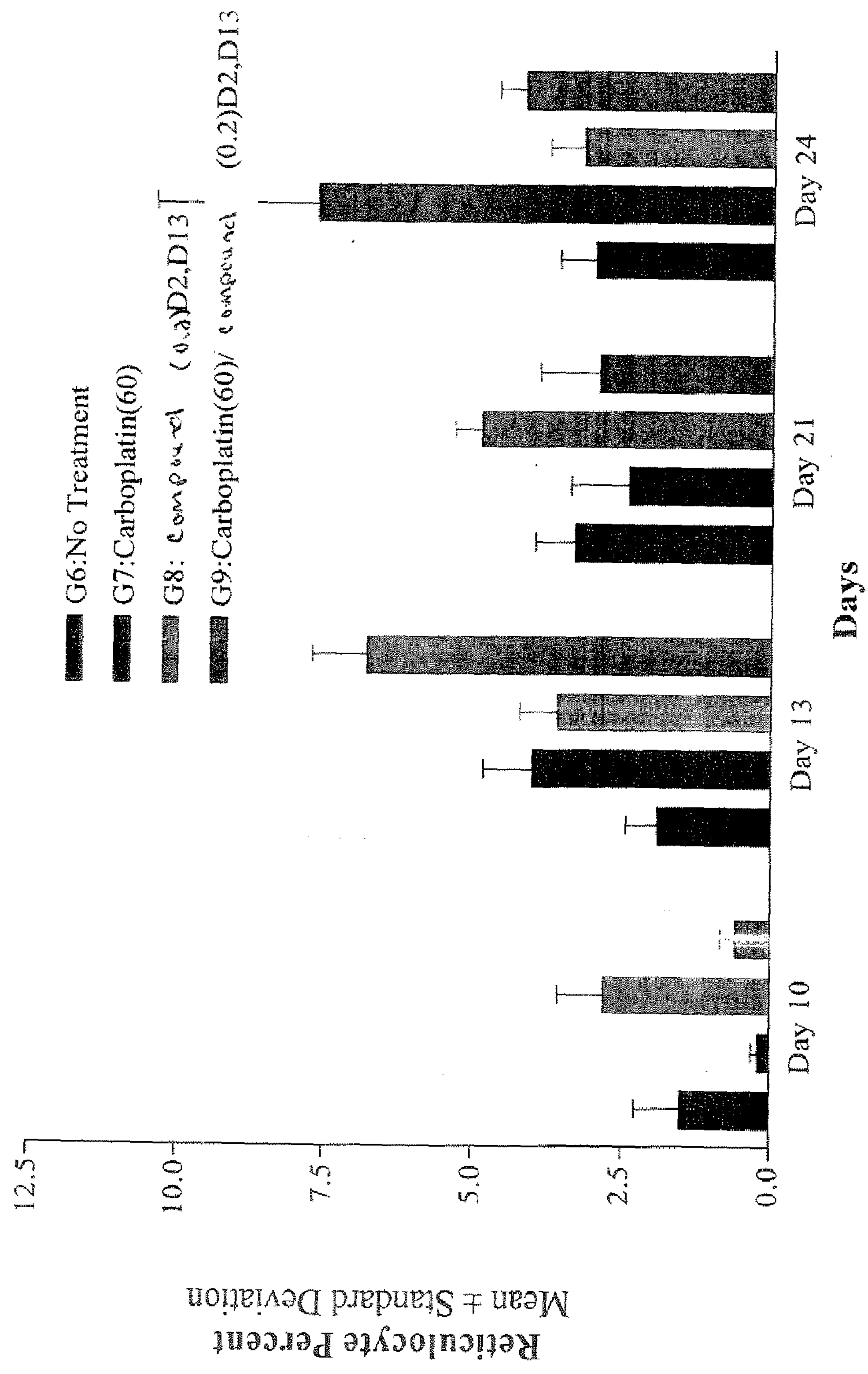

Figure 3c. Mean Hematocrit Values in the HT29-e141 Study

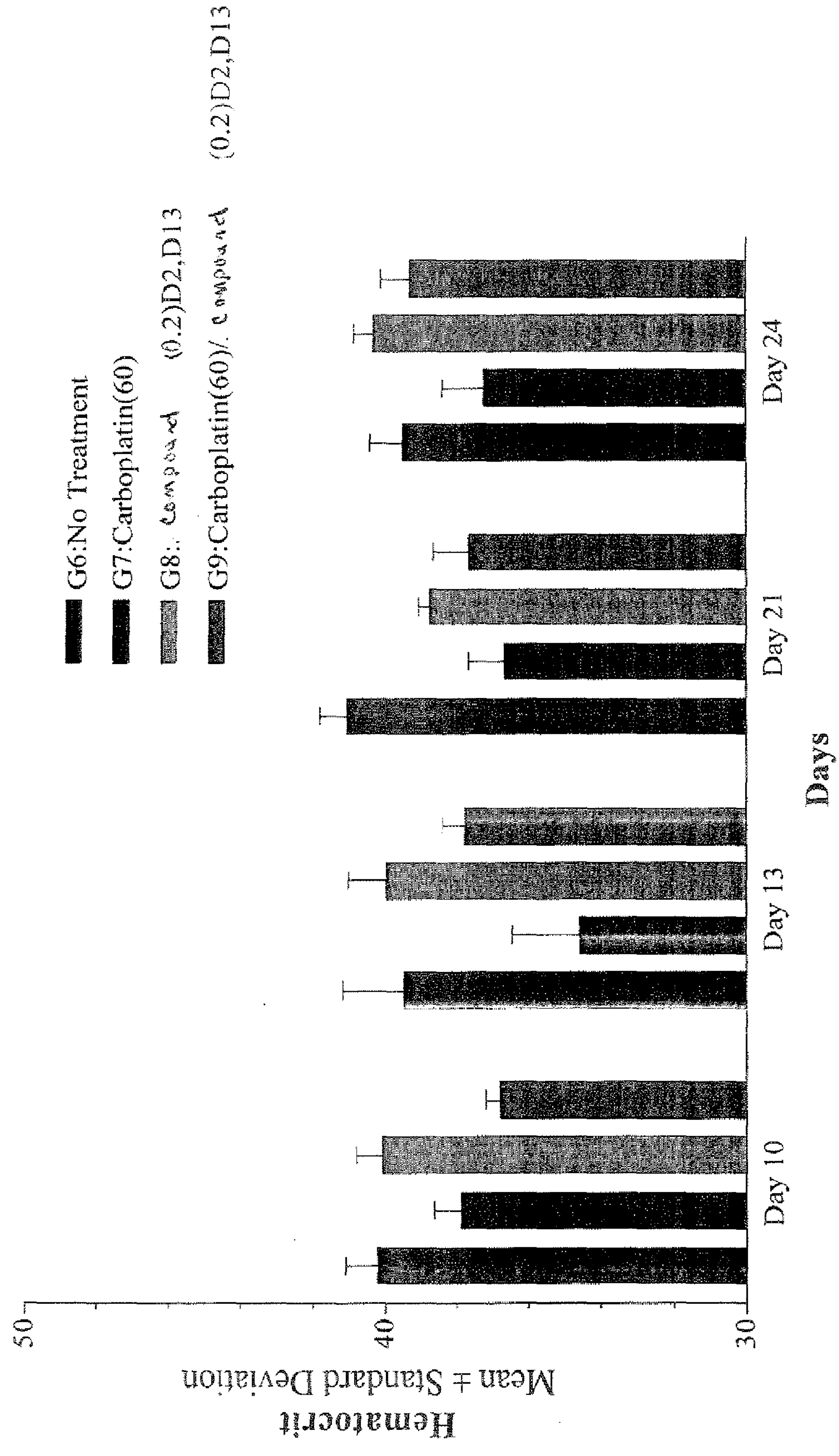

Figure 4

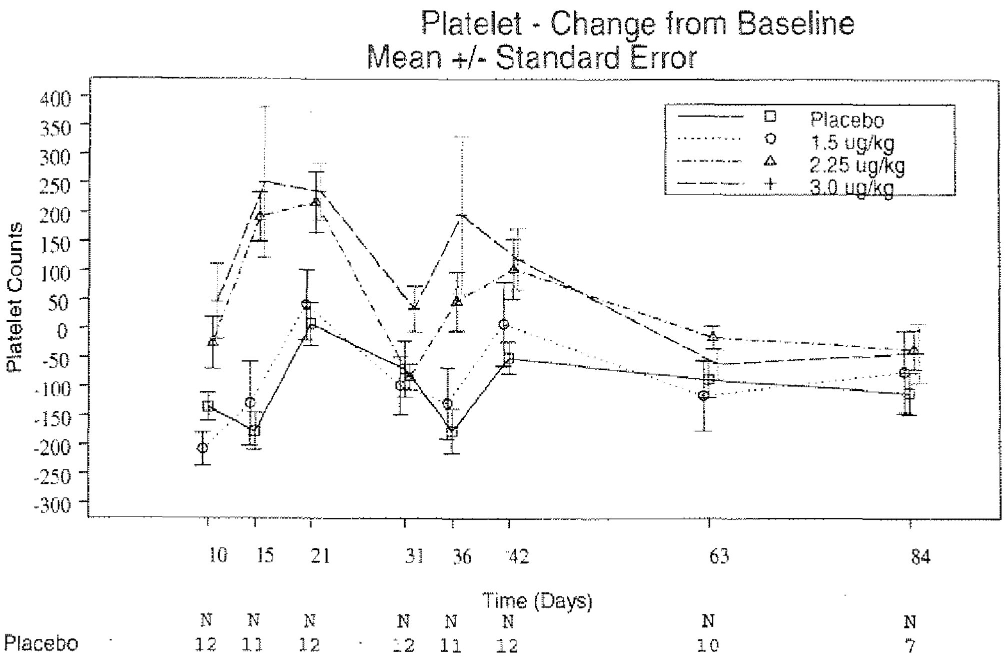


| | N | N | N | N | N | N | N | N |
|---|---|---|---|---|---|---|---|---|
| Placebo | 12 | 11 | 12 | 12 | 11 | 12 | 10 | 7 |
| 1.5 ug/kg | 9 | 9 | 8 | 7 | 7 | 7 | 6 | 5 |
| 2.25 ug/kg | 10 | 10 | 9 | 9 | 9 | 9 | 7 | 6 |
| 3.0 ug/kg | 9 | 9 | 9 | 7 | 6 | 8 | 9 | 6 |

Figure 5

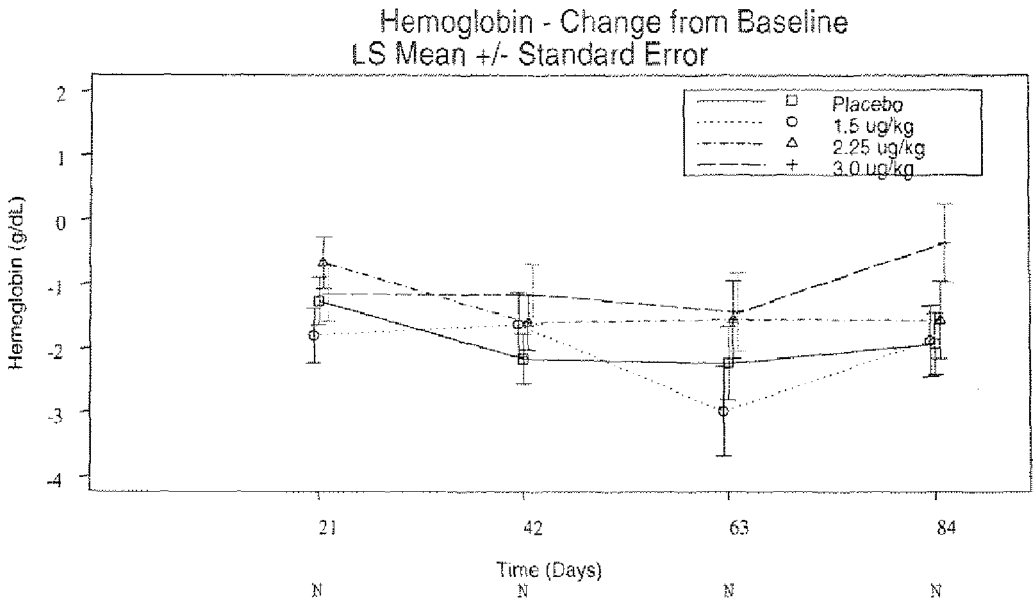


| | N | N | N | N |
|---|---|---|---|---|
| Placebo | 12 | 12 | 9 | 8 |
| 1.5 ug/kg | 9 | 7 | 6 | 6 |
| 2.25 ug/kg | 10 | 10 | 8 | 5 |
| 3.0 ug/kg | 9 | 8 | 8 | 5 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7576056 B
- US 7723295 B
- US 20080119384 A
- US 10918561 B
- US 11200416 B
- US 11354065 B
- US 91856104 A
- US 35406506 A


### Non-patent literature cited in the description


- **LUDWIG H ; VAN BELLE S ; BARRETT-LEE P ; BIRGEGARD G ; BOKEMEYER C ; GASCON P ; KOSMIDIS P ; KRZAKOWSKI M ; NORTIER J ; OLMI P.** The European cancer anaemia survey (ECAS): A large, multinational, prospective survey defining the prevalence, incidence, and treatment of anaemia in cancer subjects. *European Journal of Cancer,* 2004, vol. 40, 2293-2306
- **GROOPMAN JE ; ITRI LM.** Chemotherapy-induced anemia in adults: incidence and treatment. *J Natl Cancer Inst,* 1999, vol. 91, 1616-1634
- **OHE Y ; OHASHI Y ; KUBOTA K ; TAMURA T ; NAKAGAWA K ; NEGORO S ; NISHIWAKI Y ; SAIJO N ; ARIYOSHI Y ; FUKUOKA M.** Randomized phase III study of cisplatin plus irinotecan versus carboplatin plus paclitaxel, cisplatin plus gemcitabine, and cisplatin plus vinorelbine for advanced non-small-cell lung cancer; Four-Arm Cooperative study in Japan. *Annals of Oncology,* 2007, vol. 18, 317-323
- **SCHILLER JH ; HARRINGTON D ; BELANI CP ; LANGER C ; SANDLER A ; KROOK J ; ZHU J ; JOHNSON DH.** Comparison of Four Chemotherapy Regimens for Advanced Non-Small Cell Lung Cancer. *N Engl J Med,* 2002, vol. 346 (2), 92-98
- **WILSON J ; YAO GL ; RAFTERY J et al.** A systematic review and economic evaluation of epoetin alfa, epoetin beta, and darbepoetin alfa in anemia associated with cancer, especially that attributable to cancer treatment. *Health Technol Assess,* 2007, vol. 11 (13), 1-220
- **RIZZO JD ; LICHTEN AE ; WOOLF SH et al.** Use of epoetin in subjects with cancer: evidence-based clinical practice guidelines of the American Society of Clinical Oncology and the American Society of Hematology. *J Clin Oncol,* 2002, vol. 20, 4083-4107
- Cancer and treatment-related anemia. National Comprehensive Cancer Network, 08 August 2005
- **BOKEMEYER C ; AAPRO MS ; COURDI A et al.** EORTC guidelines for the use of erythropoietic proteins in anaemic subjects with cancer. *Eur J Cancer,* 2004, vol. 40, 2201-2216
- Clinical Practice Guideline Update on the Use of Epoetin and Darbepoetin. **RIZZO JD ; SOMERFIELD MR ; HAGERTY KL et al.** J Clin Oncol 2007. American Society of Clinical Oncology, 2007, 1-17
- **MURONE M. ; CARPENTER DA. ; DE SAUVAGE FJ.** Hematopoietic deficiencies in c-mpl and TPO knockout mice. *Stem Cells,* 1998, vol. 16, 1-6
- **BALLMAIER M. ; GERMESHAUSEN M ; SCHULZE H et al.** c-mpl mutations are the cause of congenital amegakaryocytic thrombocytopenia. *Blood,* 2001, vol. 97, 139-146
- **BORGE OJ ; RAMSFJELL V ; CUI L ; JACOBSEN SEW.** Ability of early acting cytokines to directly promote survival and suppress apoptosis of human primitive CD34+CD38-bone marrow cells with multilineage potential at the single-cell level: key role of thrombopoietin. *Blood,* 1997, vol. 90, 2282-2292
- **MOUTHON MA ; VAN DER MEEREN A ; GAULER MH ; VISSER TP ; SQUIBAN C ; GOURMELON P ; WAGEMAKER G.** Thrombopoietin promotes hematopoietic recovery and survival after high-dose whole body irradiation. *Int J Radiat Oncol Biol Phys,* 1999, vol. 43, 867-875
- **DROUET M ; MATHIEU J ; GRENIER N ; MULTON E ; SOTTO JJ ; HERODIN F.** The reduction of in vitro radiation-induced Fas-related apoptosis in CD34+ progenitor cells by SCF, FLT-3 ligand, TPO, and IL-3 in combination resulted in CD34+ cell proliferation and differentiation. *Stem Cells,* 1999, vol. 17, 273-285

- **RAMSFJELL V ; BORGE OJ ; CUI L ; JACOBSEN SE.** Thrombopoietin directly and potently stimulates multilineage growth and progenitor cell expansion from primitive (CD34+ CD38-) human bone marrow progenitor cells: distinct and key interactions with the ligands for c-kit and flt3, and inhibitory effects of TGF-beta and TNF-alpha. *J Immunol,* 1997, vol. 158, 5169-5177
- **NEELIS KJ ; VISSER TP ; DIMJATI W et al.** A single dose of thrombopoietin shortly after myelosuppressive total body irradiation prevents pancytopenia in mice by promoting short-term multilineage spleen-repopulating cells at the transient expense of bone marrow-repopulating cells. *Blood,* 1998, vol. 92, 1586-1597
- **TORII Y ; NITTA Y ; AKAHORI H et al.** Mobilization of primitive haemopoietic progenitor cells and stem cells with long-term repopulating ability into peripheral blood in mice by pegylated recombinant human megakaryocyte growth and development factor. *Br J Haematol,* 1998, vol. 103, 1172-1180
- **ELTING LS ; RUBENSTEIN EB ; MARTIN CG et al.** Incidence, cost, and outcomes of bleeding and chemotherapy dose modification among solid tumor subjects with chemotherapy-induced thrombocytopenia. *J Clin Oncol,* 2001, vol. 19, 1137-1146
- **LI J ; YANG C ; XIA Y et al.** Thrombocytopenia caused by the development of antibodies to thrombopoietin. *Blood,* 2001, vol. 98, 3241-3248
- **COLUMBYOVA L ; MASSIMA L ; SCADDEN D.T.** Thrombopoietin receptor expression in human cancer cell lines and primary tissues. *Cancer Res.,* 1995, vol. 55, 3509-3512
- *Investigator's Brochure - The TPO mimetic peptide compound,* November 2007
- **JEMAL A ; SIEGEL R ; WARD E ; MURRAY T ; XU J ; THUN MJ.** Cancer statistics. *2007. CA Cancer J Clin,* 2007, vol. 57, 43-66
- AJCC Cancer Staging Manual. Springer-Verlag, 2002
- **BELANI CP ; LAROCCA RV ; RINALDI WJ.** A multicenter, phase III randomized trial for stage IIIB/IV NSCLC of weekly paclitaxel and carboplatin vs. standard paclitaxel and carboplatin given every three weeks, followed by weekly paclitaxel. *Proc Am Soc Clin Oncol,* 2004, vol. 23, 619
- **BELANI CP ; LEE JS ; SOCINSKI MA ; ROBERT F ; WATERHOUSE D ; ROWLAND K ; ANSARI R ; LILENBAUM R ; NATALE RB.** Randomized phase III trial comparing cisplatin-etoposide to carboplatin-paclitaxel in advanced or metastatic non-small cell lung cancer. *Ann Oncol,* 2005, vol. 16, 1069-75
- **CARDENAL F ; LOPEZ-CABRERIZO MP ; ANTON A ; ALBEROLA V ; MASSUTI B ; CARRATO A ; BARNETO I ; LOMAS M ; GARCIA M ; LIANES P.** Randomized phase III study of gemcitabine-cisplatin versus etoposide-cisplatin in the treatment of locally advanced or metastatic non-small-cell lung cancer. *J Clin Oncol,* 1999, vol. 17, 12-8
- **FOSSELLA F ; PEREIRA JR ; VON PAWEL J ; PLUZANSKA A ; GORBOUNOVA V ; KAUKEL E ; MATTSON KV ; RAMLAU R ; SZCZESNA A ; FIDIAS P.** Randomized, multinational, phase III study of docetaxel plus platinum combinations versus vinorelbine plus cisplatin for advanced non-small-cell lung cancer: the TAX 326 study group. *J Clin Oncol,* 2003, vol. 21, 3016-24
- **KELLY K ; CROWLEY J ; BUNN PA, JR. ; PRESANT CA ; GREVSTAD PK ; MOINPOUR CM ; RAMSEY SD ; WOZNIAK AJ ; WEISS GR ; MOORE DF.** Randomized phase III trial of paclitaxel plus carboplatin versus vinorelbine plus cisplatin in the treatment of subjects with advanced non--small-cell lung cancer: a Southwest Oncology Group trial. *J Clin Oncol,* 2001, vol. 19, 3210-8
- **OHE Y ; OHASHI Y ; KUBOTA K ; TAMURA T ; NAKAGAWA K ; NEGORO S ; NISHIWAKI Y ; SAIJO N ; ARIYOSHI Y ; FUKUOKA M.** Randomized phase III study of cisplatin plus irinotecan versus carboplatin plus paclitaxel, cisplatin plus gemcitabine, and cisplatin plus vinorelbine for advanced non-small-cell lung cancer: Four-Arm Cooperative Study in Japan. *Ann Oncol,* 2007, vol. 18, 317-23
- **RUDD R ; GOWER N ; JAMES L.** Phase III randomised comparison of gemcitabine and carboplatin with mitomycin, ifosfamide and cisplatin in advanced non-small cell lung cancer. *Proc Am Soc Clin Oncol,* 2002, vol. 21
- **SANDLER AB ; NEMUNAITIS J ; DENHAM C ; VON PAWEL J ; CORMIER Y ; GATZEMEIER U ; MATTSON K ; MANEGOLD C ; PALMER MC ; GREGOR A.** Phase III trial of gemcitabine plus cisplatin versus cisplatin alone in subjects with locally advanced or metastatic non-small-cell lung cancer. *J Clin Oncol,* 2000, vol. 18, 122-30
- **SEDERHOLM C ; HILLERDAL G ; LAMBERG K ; KOLBECK K ; DUFMATS M ; WESTBERG R ; GAWANDE SR.** Phase III trial of gemcitabine plus carboplatin versus single-agent gemcitabine in the treatment of locally advanced or metastatic non-small-cell lung cancer: the Swedish Lung Cancer Study Group. *J Clin Oncol,* 2005, vol. 23, 8380-8

- **SMIT EF ; VAN MEERBEECK JP ; LIANES P ; DEBRUYNE C ; LEGRAND C ; SCHRAMEL F ; SMIT H ; GAAFAR R ; BIESMA B ; MANEGOLD C.** Three-arm randomized study of two cisplatin-based regimens and paclitaxel plus gemcitabine in advanced non-small-cell lung cancer: a phase III trial of the European Organization for Research and Treatment of Cancer Lung Cancer Group--EORTC 08975. *J Clin Oncol,* 2003, vol. 21, 3909-17
- **WOZNIAK AJ ; CROWLEY JJ ; BALCERZAK SP ; WEISS GR ; SPIRIDONIDIS CH ; BAKER LH ; ALBAIN KS ; KELLY K ; TAYLOR SA ; GANDARA DR.** Randomized trial comparing cisplatin with cisplatin plus vinorelbine in the treatment of advanced non-small-cell lung cancer: a Southwest Oncology Group study. *J Clin Oncol,* 1998, vol. 16, 2459-65
- **ABRATT RP ; HART GJ.** 10-year update on chemotherapy for non-small cell lung cancer. *Ann Oncol,* 2006, vol. 17 (5), v33-v36
- **SANDLER A ; GRAY R ; PERRY MC ; BRAHMER J ; SCHILLER JH ; DOWLATI A ; LILENBAUM R ; JOHNSON DH.** Paclitaxel-carboplatin alone or with bevacizumab for non-small-cell lung cancer. *N Engl J Med,* 2006, vol. 355, 2542-50
- **MANEGOLD C ; VON PAWEL J ; ZATLOUKAL P.** Randomised, double-blind multicentre phase III study of bevacizumab in combination with cisplatin and gemcitabine in chemotherapy-naive subjects with advanced or recurrent non-squamous non-small cell lung cancer (NSCLC): B017704. *Proc Am Soc Clin Oncol,* 2007, vol. 25
- **SANBORN RE ; SANDLER AB.** The safety of bevacizumab. *Expert Opin Drug Saf,* 2006, vol. 5, 289-301
- **LANGER C ; LI S ; SCHILLER J ; TESTER W ; RAPOPORT BL ; JOHNSON DH.** Randomized phase II trial of paclitaxel plus carboplatin or gemcitabine plus cisplatin in Eastern Cooperative Oncology Group performance status 2 non-small-cell lung cancer subjects: ECOG 1599. *J Clin Oncol,* 2007, vol. 25, 418-23
- **WANG LR ; HUANG MZ ; ZHANG GB ; XU N ; WU XH.** Phase II study of gemcitabine and carboplatin in subjects with advanced non-small-cell lung cancer. *Cancer Chemother Pharmacol,* 2007, vol. 60, 601-7
- **THERASSE P ; ARBUCK S ; EISENHAUER EA et al.** New guidelines to evaluate the response to treatment in solid tumors. *J Natl Cancer Inst,* 2000, vol. 92 (3), 205-16
- *RECIST guidelines,* 05 November 2007, http://ctep.cancer.gov/guidelines/recist.html
- **BOKEMEYER C ; AAPRO MS et al.** EORTC guidelines for the use of erythropoietic proteins in anaemic subjects with cancer: 2006 update. *Eur J Cancer,* 2007, vol. 43 (2), 258-70
- Cancer and Treatment Related Anemia V.3.2007. National Comprehensive Cancer Network®
- **SCHIFFER CA ; ANDERSON KC ; BENNETT CL et al.** Platelet transfusion for subjects with cancer: clinical practice guidelines of the American society of clinical oncology. *J Clin Oncol,* 2001, vol. 19 (5), 1519-38
- **OKEN, M.M. ; CREECH, R.H. ; TORMEY, D.C. ; HORTON, J. ; DAVIS, T.E. ; MCFADDEN, E.T. ; CARBONE, P.P.** Toxicity And Response Criteria Of The Eastern Cooperative Oncology Group. *Am J Clin Oncol,* 1982, vol. 5, 649-655
- **YELLEN SB ; CELLA DF ; WEBSTER K ; BLENDOWSKI C ; KAPLAN E.** Measuring fatigue and other anemia-related symptoms with the Functional Assessment of Cancer Therapy (FACT) measurement system. *J Pain Symptom Manage,* February 1997, vol. 13 (2), 63-74
- **CELLA D ; WEBSTER K.** *Linking outcomes management to quality-of-life measurement. Oncology (Williston Park),* November 1997, vol. 11 (11A), 232-5
- **MENDOZA TR ; WANG XS ; CLEELAND CS ; MORRISSEY M ; JOHNSON BA ; WENDT JKI ; HUBER SL.** The rapid assessment of fatigue severity in cancer subjects: use of the Brief Fatigue Inventory. *Am Cancer Soc,* 1999, vol. 85, 1186-96
- **CRINO L ; SCAGLIOTTI GV et al.** Gemcitabine and cisplatin versus mitomycin, ifosfamide, and cisplatin in advanced non-small-cell lung cancer: a randomized phase III study of the Italian lung cancer project. *J Clin Oncol,* 1999, vol. 17 (11), 3522-30
- **CELLA D ; ETON DT ; LAI JIN-SHEI ; PETERMAN AH ; MERKEL DE.** Combining Anchor and distribution-based methods to derive minimal clinically important differences on the Functional Assessment of Cancer Therapy (FACT) Anemia and Fatigue Scales. *J Pain and Symptom Management,* 2002, vol. 24 (6), 547-561
- **LOKICH J ; ANDERSON N.** Carboplatin versus cisplatin in solid tumors: An analysis of the literature. *Annals of Oncology,* 1998, vol. 9, 13-21
- Cancer Therapy Evaluation Program. Common Terminology Criteria for Adverse Events Version 3.0. NIH, 2006, 1-72